(19) Europäisches Patentamt
European Patent Office
Office européen des brevets

(11) Veröffentlichungsnummer: **0 177 960 B1**

(12) # EUROPÄISCHE PATENTSCHRIFT

(45) Veröffentlichungstag der Patentschrift: **20.03.91**

(51) Int. Cl.$^{5}$: **C07C 215/42**, C07C 217/52, C07C 217/00, C07C 323/30, A61K 31/135, A61K 31/215

(21) Anmeldenummer: **85112863.7**

(22) Anmeldetag: **10.10.85**

(54) **Tetrahydronaphtalinderivate, Verfahren und Zwischenprodukte zu deren Herstellung sowie diese enthaltende Arzneimittel.**

(30) Priorität: **11.10.84 CH 4870/84**

(43) Veröffentlichungstag der Anmeldung:
**16.04.86 Patentblatt 86/16**

(45) Bekanntmachung des Hinweises auf die Patenterteilung:
**20.03.91 Patentblatt 91/12**

(84) Benannte Vertragsstaaten:
**AT BE CH DE FR GB IT LI LU NL SE**

(56) Entgegenhaltungen:
**EP-A- 0 080 721**

(73) Patentinhaber: **F. HOFFMANN-LA ROCHE AG**
**Postfach 3255**
**CH-4002 Basel(CH)**

(72) Erfinder: **Hengartner, Urs, Dr.**
**Binzenstrasse 28**
**CH-4058 Basel(CH)**
Erfinder: **Ramuz, Henri, Prof. Dr.**
**Rheinparkstrasse 3**
**CH-4127 Birsfelden(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

EP 0 177 960 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Tetrahydronaphthalinderivate. Im speziellen betrifft sie Tetrahydronaphthalinderivate der allgemeinen Formel

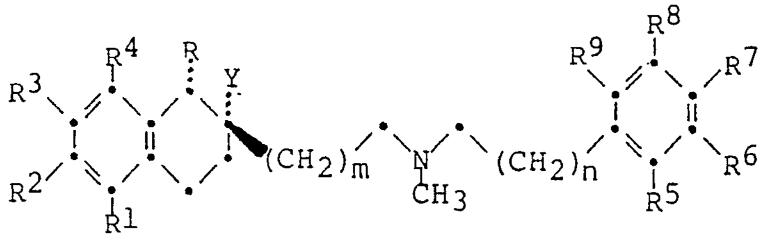

I

worin R Wasserstoff oder nieder-Alkyl, $R^1$, $R^2$, $R^3$ und $R^4$ unabhängig voneinander je Wasserstoff, Halogen oder nieder-Alkoxy oder zwei benachbarte Reste zusammen Methylendioxy oder Aethylendioxy, $R^5$, $R^6$, $R^7$, $R^8$ und $R^9$ unabhängig voneinander je Wasserstoff, Halogen, $C_1$-$C_{10}$-Alkoxy, nieder Alkylthio, nieder-Alkoxy-nieder-alkoxy oder ω,ω,ω-Trifluor-nieder-alkoxy oder zwei benachbarte Reste zusammen Methylendioxy oder Aethylendioxy, Y Hydroxy, nieder-Alkylcarbonyloxy, nieder-Alkoxy-nieder-alkylcarbonyloxy, nieder-Alkoxycarbonyloxy, nieder-Alkoxy-nieder-alkoxycarbonyloxy, nieder-Alkylthio-nieder-alkylcarbonyloxy oder gegebenen falls substituiertes Benzylcarbonyloxy, m die Zahl 1 oder 2 und n die Zahl 1, 2 oder 3 bedeuten, in Form von Racematen und optischen Antipoden, sowie pharmazeutisch verwendbare Säureadditionssalze davon.

Diese Verbindungen sind neu und zeichnen sich durch wertvolle pharmakodynamische Eigenschaften aus.

Gegenstand der vorliegenden Erfindung sind Verbindungen der allgemeinen Formel I als solche und zur Anwendung als therapeutische Wirkstoffe, die Herstellung dieser Verbindungen. Zwischenprodukte für die Herstellung dieser Verbindungen, ferner diese enthaltende Arzneimittel und die Herstellung solcher Arzneimittel, sowie die Verwendung von Verbindungen der allgemeinen Formel I bei der Bekämpfung bzw. Verhütung von Krankheiten bzw. bei der Verbesserung der Gesundheit, insbesondere bei der Bekämpfung bzw. Verhütung von Angina pectoris, Ischämie, Arrhythmien und Bluthochdruck

Angaben zum Stand der Technik

In EPA 0.080.721 sind Phenylalkylamine und deren koronargefässerweiternde, antihypoxische, antispasmodische und Blutblättchenagregations-hemmende Eigenschaften bekannt. Die anmeldegemässen Verbindungen unterscheiden sich durch die Anwesenheit einer die Alkylenkette unterbrechenden Aminogruppe sowie den Ersatz des Phenylrings durch einen Tetrahydronaphthalinrest in signifikanter Weise von den vorbekannten Verbindungen.

Der in der vorliegenden Beschreibung verwendete Ausdruck "nieder-Alkyl" - allein oder in Kombination - bedeutet geradkettige und verzweigte, gesättigte Kohlenwasserstoffreste mit 1-6, vorzugsweise 1-4, Kohlenstoffatomen, wie Methyl, Aethyl, n-Propyl, Isopropyl, n-Butyl, Isobutyl, sec.-Butyl, tert.-Butyl und dergleichen. Der Ausdruck "nieder-Alkoxy" bedeutet nieder-Alkyläthergruppen, worin der Ausdruck "nieder-Alkyl" die obige Bedeutung hat. Der Ausdruck "$C_1$-$C_{10}$-Alkoxy" betrifft in ähnlicher Weise Alkyläthergruppen, worin der Alkylrest 1-10 Kohlenstoffatome aufweist. Der Ausdruck "Halogen" umfasst die vier Halogenatome Fluor, Chlor, Brom und Jod. Der Ausdruck "substituiertes Benzylcarbonyloxy" umfasst Benzylcarbonyloxy-Gruppen, in denen ein oder zwei Wasserstoffatome durch nieder-Alkyl, nieder--Alkoxy, Halogen oder Nitro substituiert sind. Der Ausdruck "Abgangsgruppe" bedeutet bekannte Gruppen wie Halogen, vorzugsweise Chlor oder Brom, Arylsulfonyloxy, wie etwa Tosyloxy, Brombenzolsulfonyloxy, Benzolsulfonyloxy oder Mesitylensulfonyloxy, oder Alkylsulfonyloxy, wie etwa Mesyloxy oder Trifluormethylsulfonyloxy.

Bevorzugt sind solche Verbindungen der Formel I, worin R nieder-Alkyl, besonders bevorzugt Isopropyl, bedeutet. Y bedeutet vorzugsweise Hydroxy, nieder-Alkylcarbonyloxy, besonders bevorzugt Acetyloxy, oder nieder-Alkoxy-nieder-alkylcarbonyloxy, besonders bevorzugt Methoxyacetyloxy. m und n bedeuten vorzugsweise die Zahl 1. Weiter sind solche Verbindungen der Formel I bevorzugt, worin zwei der Substituenten $R^1$, $R^2$, $R^3$ und $R^4$ Wasserstoff und die beiden anderen unabhängig voneinander je Wasserstoff, Halogen oder nieder-Alkoxy bedeuten. Besonders bevorzugt sind solche Verbindungen der Formel I, worin $R^1$ und

$R^4$ je Wasserstoff und $R^2$ und $R^3$ je nieder-Alkoxy oder $R^2$ Halogen und $R^3$ Wasserstoff bedeuten. Ebenfalls bevorzugt sind die Verbindungen der Formel I, worin zwei der Substituenten $R^5$, $R^6$ , $R^7$ , $R^8$ und $R^9$ Wasserstoff und die drei anderen unabhängig voneinander je Wasserstoff, Halogen, $C_1$-$C_{10}$-Alkoxy, nieder-Alkylthio oder nieder-Alkoxy-nieder-alkoxy bedeuten. Besonders bevorzugt bedeuten $R^8$ und $R^9$ je Wasserstoff und $R^5$ Wasserstoff oder $C_1$-$C_{10}$-Alkoxy, $R^6$ Wasserstoff, $C_1$-$C_{10}$-Alkoxy oder nieder-Alkoxy-nieder-alkoxy und $R^7$ Wasserstoff, Halogen, $C_1$-$C_{10}$-Alkoxy, nieder-Alkylthio oder nieder-Alkoxy-nieder-alkoxy.

Aus dem obigen folgt, dass solche Verbindungen der Formel I ganz besonders sind, worin R Isopropyl, Y Hydroxy, Acetyloxy oder Methoxyacetyloxy, $R^1$, $R^4$, $R^8$ und $R^9$ je Wasserstoff, $R^2$ und $R^3$ je nieder-Alkoxy oder $R^2$ Halogen und $R^3$ Wasserstoff, $R^5$ Wasserstoff oder $C_1$-$C_{10}$-Alkoxy, R6 Wasserstoff, $C_1$-$C_{10}$-Alkoxy oder nieder-Alkoxy-nieder-alkoxy, $R^7$ Wasserstoff, Halogen, $C_1$-$C_{10}$-Alkoxy, nieder-Alkylthio oder nieder-Alkoxy-nieder-alkoxy und m und n die Zahl 1 bedeuten. Ganz speziell bevorzugte Verbindungen der Formel I sind:

2-[2-[(3,4-Dimethoxyphenäthyl)methylamino]äthyl]-6-fluor-1,2,3,4-tetrahydro-1α-isopropyl-2α-naphthylmethoxyacetat,

6.7-Dimethoxy-2-[2-[(3,4-dimethoxyphenäthyl)methylamino] äthyl]-1,2,3,4-tetrahydro-1α-isopropyl-2α-naphthylmethoxyacetat,

6.7-Dimethoxy-2-[2-[(3.4-dimethoxyphenäthyl)methylamino] äthyl]-1,2,3,4-tetrahydro-1α-isopropyl-2α-naphthylacetat und

[1S,2S]-2-[2-[(3.4-Dimethoxyphenäthyl)methylamino]äthyl]-6-fluor-1,2,3,4-tetrahydro-1-isopropyl-2-naphthylmethoxyacetat.

Die Verbindungen der Formel I in Form von Racematen und optischen Antipoden, sowie pharmazeutisch verwendbare Säureadditionssalze davon können hergestellt werden, indem man

a) zur Herstellung von Verbindungen der Formel I, worin Y Hydroxy bedeutet und die übrigen Symbole die oben angegebene Bedeutung besitzen, eine Verbindung der allgemeinen Formel

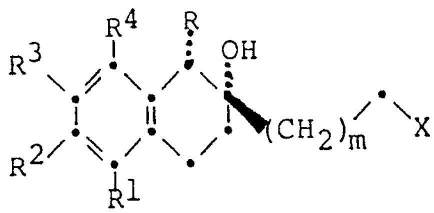

II

worin x eine Abgangsgruppe bedeutet und R, $R^1$, $R^2$, $R^3$, $R^4$ und m die oben angegebene Bedeutung besitzen. mit einem Amin der allgemeinen Formel

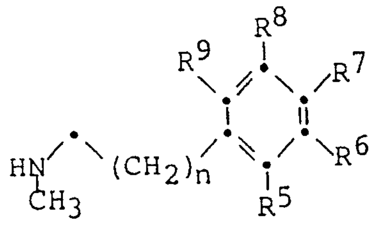

III

worin $R^5$ , $R^6$ , $R^7$ , $R^8$ , $R^9$ und n die oben angegebene Bedeutung besitzen, umsetzt, oder

b) zur Herstellung von Verbindungen der Formel I, worin Y Hydroxy und m die Zahl 2 bedeuten und die übrigen Symbole die oben angegebene Bedeutung besitzen, einen Aldehyd der allgemeinen Formel

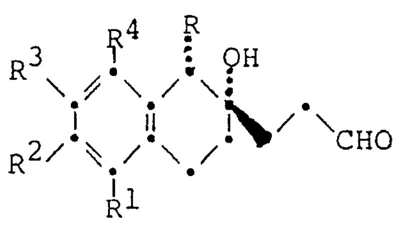

IV

worin R, $R^1$, $R^2$, $R^3$ und $R^4$ die oben angegebene Bedeutung besitzen, mit einem Amin der obigen Formel III reduktiv aminiert, oder

c) zur Herstellung von Verbindungen der Formel I, worin Y nieder-Alkylcarbonyloxy, nieder-Alkoxy-nieder-alkylcarbonyloxy, nieder-Alkoxycarbonyloxy, nieder-Alkoxy-nieder-alkoxycarbonyloxy, nieder-Alkylthio-nieder-alkylcarbonyloxy oder gegebenenfalls substituiertes Benzylcarbonyloxy bedeutet und die übrigen Symbole die oben angegebene Bedeutung besitzen, eine Verbindung der allgemeinen Formel

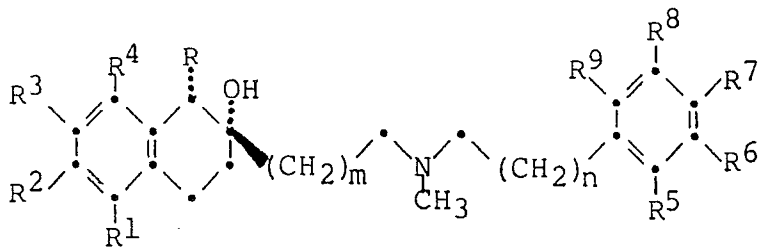

Ia

worin R, $R^1$, $R^2$, $R^3$, $R^4$, $R^5$, $R^6$, $R^7$, $R^8$, $R^9$ , m und n die oben angegebene Bedeutung besitzen, mit einem eine nieder-Alkylcarbonyl-, nieder-Alkoxy-nieder--alkylcarbonyl-, nieder-Alkoxycarbonyl-, nieder-Alkoxy-nieder-alkoxycarbonyl-, nieder-Alkylthio-nieder-alkylcarbonyl-oder gegebenenfalls substituierte Benzylcarbonyl -Gruppe abgebenden Acylierungsmittel umsetzt, und

d) erwünschtenfalls, ein erhaltenes Racemat in die optischen Antipoden auftrennt, und/oder

e) eine erhaltene Verbindung in ein pharmazeutisch verwendbares Säureadditionssalz überführt.

Eine Verbindung der Formel II wird nach an sich bekannten Methoden mit einem Amin der Formel III umgesetzt. Die Umsetzung erfolgt in Gegenwart oder Abwesenheit eines unter den Reaktionsbedingungen inerten organischen Lösungsmittels bei einer Temperatur zwischen etwa 20° und 150° C, vorzugsweise zwischen etwa 80ü und 120° C. Bei dieser Umsetzung kommen Lösungsmittel, wie Dimethylformamid, Dimethylsul-foxid, Alkohole, wie Isopropanol oder tert.-Butanol, Aether, wie Tetrahydrofuran oder Dioxan, aromatische Kohlenwasserstoffe, wie Benzol, Toluol oder Xylol, chlorierte Kohlenwasserstoffe, wie Methylenchlorid, Tetrachlorkohlenstoff oder Chlorbenzol, und dgl. in Frage. Die Umsetzung erfolgt vorteilhafterweise in Gegenwart eines säurebindenden Mittels, wie eines tertiären Amins, wie Trimethylamin, Triäthylamin, Aethyldiisopropylamin oder 1,5-Diazabicyclo[4.3.0]non-5-en, wobei auch überschüssiges Amin der Formel III als säurebindendes Mittel dienen kann. Aus Zweckmässigkeitsgründen arbeitet man bei Atmosphärendruck, obwohl höhere Drucke ebenfalls angewandt werden können.

Die reduktive Aminierung einer Verbindung der Formel IV erfolgt ebenfalls in an sich bekannter Weise durch Umsetzen mit einem Amin der Formel III in Gegenwart eines Reduktionsmittels, wie Natriumcyanoborhydrid oder Wasserstoff/Katalysator, in einem Lösungsmittel, wie einem Alkohol oder einem wässrigen Alkohol, wie Methanol, Aethanol oder Propanol oder wässrigen Lösungen davon. Aus Zweckmässigkeitsgründen erfolgt die reduktive Aminierung bei Atmosphärendruck, obwohl insbesondere bei der Verwendung von Wasserstoff/Katalysator als Reduktionsmittel auch bei höheren Drucken gearbeitet werden kann. Geeignete Katalysatoren sind insbesondere Raney-Nickel und Palladium. Die Reaktionstemperatur liegt in Abhängigkeit vom eingesetzten Reduktionsmittel zwischen etwa 0° und 50° C für Natriumcyanoborhydrid und zwischen etwa 0° und 100° C für Wasserstoff/Katalysator.

Die Acylierung einer Verbindung der Formel Ia erfolgt ebenfalls nach an sich bekannten Methoden. Geeignete Acylierungsmittel sind insbesondere aktivierte Säurederivate, wie Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0° C und der Rückflusstemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dgl. in Frage.

Die Ausgangsstoffe der Formeln II und IV sind neu und ebenfalls Gegenstand der vorliegenden Erfindung. Ein Herstellungsverfahren ist im nachfolgenden Schema I skizziert. Bezüglich der genauen Reaktionsbedingungen wird auf den Beispielteil verwiesen.

Schema I

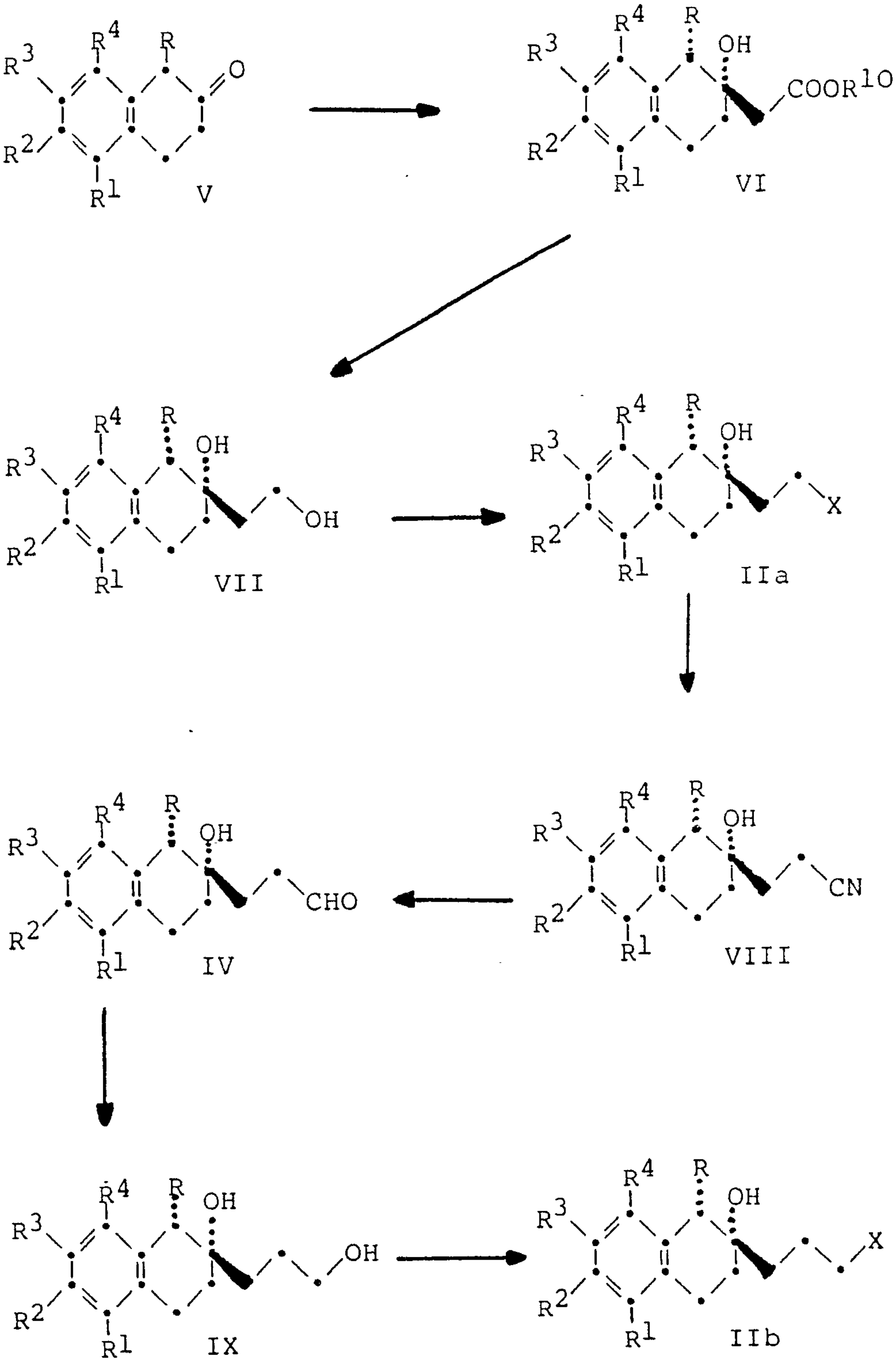

V VI VII IIa VIII IV IX IIb

worin R, $R^1$, $R^2$, $R^3$, $R^4$ und X die oben angegebene Bedeutung besitzen und $R^{10}$ Wasserstoff oder nieder-Alkyl bedeutet.

In der ersten Stufe wird in an sich bekannter Weise ein Tetralonderivat der Formel V mit einem Halogenessigsäure-alkylester in Gegenwart von Zink umgesetzt, wobei die Umsetzung mit einem Halogenessigsäure-tert.-butylester auch in Gegenwart von Magnesium durchgeführt werden kann. Die Umsetzung erfolgt in einem inerten organischen Lösungsmittel oder Lösungsmittelgemisch, wie einem Aether, wie Diäthyläther oder Tetrahydrofuran, oder einem aromatischen Kohlenwasserstoff, wie Benzol oder Toluol, oder Gemischen davon bei einer Temperatur zwischen etwa 0° C und der Rückflusstemperatur des Lösungsmittels. In situ-Zerlegung des intermediär gebildeten Additionsproduktes liefert einen Ester der

Formel VI, welcher in an sich bekannter Weise zur entsprechenden Säure der Formel VI hydrolysiert werden kann.

Ein Ester oder eine Säure der Formel VI kann ebenfalls nach bekannten Methoden zum entsprechenden Alkohol der Formel VII reduziert werden. Geeignete Reduktionsmittel sind beispielsweise Lithiumaluminiumhydrid, Natrium-bis-(2-methoxyäthoxy)aluminiumdihydrid, Lithiumborhydrid, Diisobutylaluminiumhydrid oder Diboran und dgl. Die Reduktion wird in einem unter den Reaktionsbedingungen inerten organischen, aprotischen Lösungsmittel, wie einem Aether, wie Diäthyläther, Tetrahydrofuran oder Dioxan, einem Kohlenwasserstoff, wie Hexan oder Cyclohexan, oder einem aromatischen Kohlenwasserstoff, wie Benzol oder Toluol, und dgl. bei einer Temperatur zwischen etwa Raumtemperatur und 100 °C, vorzugsweise zwischen etwa Raumtemperatur und 50 °C, durchgeführt.

Ein Alkohol der Formel VII kann dann durch Umsetzen mit einem Aryl- oder Alkylsulfonylhalogenid in bekannter Weise in eine Verbindung der Formel IIa überführt werden, worin X eine Aryl- oder Alkylsulfonylgruppe bedeutet. Diese Umsetzung erfolgt vorteilhafterweise in Gegenwart eines säurebindenden Mittels, wie eines tertiären Amins, wie Triäthylamin, Aethyldiisopropylamin oder Pyridin, in Gegenwart oder Abwesenheit eines unter den Reaktionsbedingungen inerten organischen Lösungsmittels bei einer Temperatur zwischen etwa 0° und 80 °c. Als Lösungsmittel kommen Aether, wie Diäthyläther, Tetrahydrofuran oder Dioxan, aromatische Kohlenwasserstoffe, wie Benzol oder Toluol, chlorierte Kohlenwasserstoffe, wie Methylenchlorid oder Chloroform, und dgl., Gemische davon oder überschüssiges säurebindendes Mittel in Frage. Eine Verbindung der Formel IIa, worin X Halogen bedeutet, erhält man zweckmässig durch Umsetzen einer Verbindung der Formel IIa, worin X Aryl- oder Alkylsulfonyl bedeutet, mit einem Natriumhalogenid in Aceton oder einem Pyridinhydrohalogenid bei einer Temperatur zwischen etwa 0° und 100 °C.

Eine Verbindung der Formel IIa wird dann nach bekannten Methoden durch Umsetzen mit Natrium- oder Kaliumcyanid in eine Verbindung der Formel VIII überführt. Die Umsetzung mit dem Cyanid wird in einem unter den Reaktionsbedingungen inerten organischen Lösungsmittel, wie Dimethylsulfoxid, Dimethylformamid, Hexamethylphosphorsäuretriamid und dgl. bei einer Temperatur zwischen etwa Raumtemperatur und 150 °C, vorzugsweise zwischen etwa 40° und 80 °C, durchgeführt.

Eine Verbindung der Formel VIII kann danach nach bekannten Methoden zu einer Verbindung der Formel IV reduziert werden. Für diese Stufe geeignete Reduktionsmittel sind beispielsweise Natrium-bis-(2-methoxyäthoxy)aluminiumdihydrid, Diisobutylaluminiumhydrid und dgl. Die Reduktion wird in einem unter den Reaktionsbedingungen inerten organischen Lösungsmittel, wie einem chlorierten Kohlenwasserstoff, wie Methylenchlorid, einem Aether, wie Diäthyläther, Tetrahydrofuran oder Dioxan, einem Kohlenwasserstoff, wie Hexan oder Cyclohexan, und dgl. bei einer Temperatur zwischen etwa 20° und 100 °C, vorzugsweise zwischen etwa 30° und 50 °C, durchgeführt.

Eine Verbindung der Formel IV kann dann weiter zum entsprechenden Alkohol der Formel IX reduziert werden, der dann seinerseits in analoger Weise, wie oben für die Ueberführung VII→IIa angegeben, in die Verbindung der Formel IIb überführt werden kann. Die Reduktion einer Verbindung der Formel IV erfolgt in an sich bekannter Weise mit für eine solche Reduktion üblichen Reduktionsmitteln, wie Wasserstoff in Gegenwart eines Katalysators, wie Raney-Nickel oder Palladium, Lithiumaluminiumhydrid, Natriumborhydrid oder Lithiumborhydrid und dgl., in einem unter den Reaktionsbedingungen inerten organischen Lösungsmittel, wie einem Aether, wie Diäthyläther oder Tetrahydrofuran, einem Alkohol, wie Methanol oder Aethanol, und dgl. bei einer Temperatur zwischen etwa 20° und 100 °C.

Die Verbindungen der Formeln VI, VII, VIII und IX sind neu, während die Tetralonderivate der Formel V entweder bekannt sind oder in Analogie zur Herstellung der bekannten Verbindungen erhalten werden können. Die Verbindungen der Formels VII und IX sind ebenfalls Gegenstand der vorliegenden Erfindung.

Die Verbindungen der allgemeinen Formel I enthalten mindestens ein asymmetrisches Zentrum (2-Stellung) und können deshalb als optische Antipoden oder als Racemate vorliegen. Verbindungen der Formel I, die mehr als ein asymmetrisches Zentrum enthalten, liegen in der durch die Formel I angegebenen relativen Konfiguration vor.

Die Verbindungen der Formel I besitzen eine ausgeprägte Calcium-antagonistische Wirkung und können deshalb als Arzneimittel verwendet werden, insbesondere für die Bekämpfung bzw. Verhütung von Angina pectoris, Ischämie, Arrhythmien und Bluthochdruck.

Die Calcium-antagonistische Wirkung sowie die blutdrucksenkenden Eigenschaften der erfindungsgemässen Verbindungen können in den nachstehend beschriebenen Tests gezeigt werden:

A. $^{3}H$-Nifedipin-Bindungsbestimmungen:

Die Bestimmung wird an Homogenaten oder an partiell-gereinigten Membranen vom Kaninchen- oder

Meerschweinchen-Herz durchgeführt. Die Reaktionsmischung (0,3 ml) besteht aus 0,2-0,8 mg Membranprotein- 1 nM $^3$H-Nifedipin (oder 0,25 nM $^3$H-Nitrendipin) und verschiedenen Konzentrationen der Prüfsubstanzen. Die Inkubation dauert 30 Minuten bei 25° C oder 37° C und wird durch Verdünnung mit dem Inkubationspuffer gestoppt; anschliessend erfolgt eine Filtration. Die filtergebundene Radioaktivität wird mit einem Szintillationszähler gemessen. Spezifische Bindung (d.h. rezeptorgebundene) wird als die Differenz zwischen total- und unspezifischgebundener Radioaktivität definiert. Die unspezifische Bindung wird in Gegenwart von einem Ueberschuss nichtradioaktivem Nifedipin (1µM) bestimmt.

Die Wirksamkeit (Potenz) einer Verbindung in diesem Test wird durch die $IC_{50}$- und % Maximale Hemmungs-Werte (% max. Inhibition) definiert. $IC_{50}$ ist die Substanzkonzentration (in Mol/1), die eine halbmaximale Inhibition der spezifischen $^3$H-Nifedipin (bzw. $^3$H-Nitrendipin)-Bindung verursacht. Die maximale Hemmung der spezifischen Bindung wird durch den % Maximale-Hemmungs-Wert angegeben; für die Referenzverbindung Nifedipin ist dieser Wert als 100% festgesetzt. Beide Parameter werden aus einer Konzentration-Bindung-Kurve extrapoliert.

B. Hunde-Koronararterienstreifen:

In diesem Versuch werden spirale Streifen (2-2,5 mm breit und 10 mm lang) von Hunde-Koronararterien geschnitten und in einer 0rgankammer unter 1,5 g Vorspannung aufgehängt. Diese Streifen werden ca. 1 bis 2 Stunden lang in Krebs-Henseleit-Pufferlösung vorinkubiert, die bei 37° C mit Oxycarbon (Gemisch von 95% Sauerstoff und 5% Kohlendioxyd) begast wird. Anschliessend wird die relaxierende Wirkung einer dieser Substanzen auf einer KCl (84`7 mM)-Kontraktur geprüft, durch Zugabe steigender Konzentrationen der Prüfsubstanz in die Organkammer. Die Calcium-Kanal-blockierende Wirkung der Prüfsubstanzen kann deshalb festgestellt werden, weil die KCl-Kontraktur ausschliesslich mittels Calcium-Einstrom durch den spannungsabhängigen Calcium-Kanal erfolgt.

Die Wirksamkeit einer Prüfsubstanz in diesem Test wird durch den $IC_{50}$-Wert angegeben. Dieser Wert wird als die Substanzkonzentration (in Mol/1) definiert, die eine halb-maximale Relaxation einer KCl-Kontraktur verursacht. Dieser Wert wird auch aus der resultierenden Konzentration-Wirkung-Kurve extrapoliert.

C. Hämodynamische Parameter am narkotisierten Hund:

Die 4 wichtigsten Messparameter (mit resp. Messeinheiten) der hämodynamischen Versuche sind: (1) CBF: Coronary Blood Flow (in ml/min) - die Blutflussgeschwindigkeit durch die Koronararterien; (2) HR: Heart Rate (in Schläge/min) - die Herzfrequenz; (3) BP: Blood Pressure (in mm Hg) - der Blutdruck; und (4) dp/dt: Rate of increase in left ventricular pressure (in mm Hg/sec) - die Anstiegsgeschwindigkeit des linksventrikulären Druckes, als Mass für die Kontraktilitätskraft des Herzens. Die Werte werden als % maximale Aenderung vom Ausgangswert (Δ%) pro verabreichter Dosis angegeben.

Dadurch bekommt man nicht nur ein Gesamtbild der Substanzwirkung, sondern auch eine Abschätzung über die potentielle Selektivität für einen bestimmten Teil des Kreislaufsystems im ganzen Organismus. Nach Verabreichung eines Anästhetikums, wird der Hund intubiert und künstlich beatmet. Blut pH, $pCO_2$, $pO_2$ und Hämoglobin werden mit einem Blut-Gas-Analysator stündlich gemessen. Der Blutdruck (systolisch und diastolisch) wird mit einer Sonde in der Aorta abdominalis gemessen. Die Herzfrequenz wird mittels eines Tachometers erfasst, der vom Druckpuls ausgelöst wird. Für die anderen Messungen muss das Herz zuerst freigelegt werden, um eine Sonde in den linken Ventrikel (Herzkammer) für die Druckmessungen (dp/dt) einsetzen zu können. Der Coronarblutfluss wird mit einer Fluss-Sonde an der linken Coronararterie (descendens) gemessen.

Die in diesen Tests erhaltenen Resultate sind in der folgenden Tabelle zusammengefasst:

Tabelle

| Verbindung | A | | B | C | | | | |
|---|---|---|---|---|---|---|---|---|
| | $IC_{50}$ [M] | % max. Inhib. | $IC_{50}$ [M] | CBF Δ % | HR Δ % | BP Δ % | dp/dt Δ % | Dosis mg/kg i.v. |
| A | $5,6 \cdot 10^{-8}$ | 77 | $2,8 \cdot 10^{-9}$ | + 93 | -3 | -18 | -5 | 0,1 |
| B | $5,0 \cdot 10^{-9}$ | 100 | $6,1 \cdot 10^{-9}$ | +100 | -20 | -23 | +13 | 0,1 |
| C | $7,0 \cdot 10^{-6}$ | 73 | $7,8 \cdot 10^{-8}$ | + 58 | -5 | -16 | +26 | 0,1 |
| D | $5,7 \cdot 10^{-7}$ | 82 | $8,7 \cdot 10^{-8}$ | + 58 | -15 | - 9 | +3 | 0,1 |
| E | $1,6 \cdot 10^{-7}$ | 88 | $2,0 \cdot 10^{-8}$ | + 65 | -7 | -12 | +22 | 0,1 |

A = 2-[2-[(3,4-Dimethoxyphenäthyl)methylamino]äthyl]-6-fluor-1,2,3,4-tetrahydro-1α-isopropyl-2α-naphthylmethoxyacetat

B = [1S,2S]-2-[2-[(3,4-Dimethoxyphenäthyl)methylamino]äthyl]-6-fluor-1,2,3,4-tetrahydro-1-isopropyl-2-naphthylmethoxyacetat

C = [1R,2R]-2-[2-[(3,4-Dimethoxyphenäthyl)methylamino]äthyl]-6-fluor-1,2,3,4-tetrahydro-1-isopropyl-2-naphthylmethoxyacetat

D = 6,7-Dimethoxy-2-[2-[(3,4-dimethoxyphenäthyl)methylamino]äthyl]-1,2,3,4-tetrahydro-1α-isopropyl-2α-naphthylacetat

E = 6,7-Dimethoxy-2-[2-[(3,4-dimethoxyphenäthyl)methylamino]äthyl]-1,2,3,4-tetrahydro-1α-isopropyl-2α-naphthylmethoyacetat

Die Verbindungen der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Verbindungen der Formel I mit pharmazeutisch inerten, anorganischen oder organischen Excipientien verarbeitet werden. Als solche Excipientien kann man z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man Verbindungen der allgemeinen Formel I bei der Bekämpfung bzw. Verhütung von Angina pectoris, Ischämie, Arrhythmien und Bluthochdruck verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 25 bis 150 mg einer Verbindung der allgemeinen Formel I angemessen sein, wobei aber die soeben angegebene obere Grenze auch überschritten werden kann, wenn sich dies als angezeigt erweisen sollte.

Die folgenden Beispiele sollen die vorliegende Erfindung erläutern, sie jedoch in keiner Weise einschränken. Alle Temperaturen sind in Celsius Graden angegeben.

Beispiel 1

(A) 20,0 g (49,2 mMol) 2-(6-Fluor-1,2,3,4-tetrahydro -2-hydroxy-1α-isopropyl-2β-naphthyl)äthyl -p-toluolsulfonat und 19,2 g (98,4 mMol) N-Methylhomoveratrylamin werden in 70 ml wasserfreiem Dimethylsulfoxid gelöst und bei 80° 1 Stunde gerührt. Das Reaktionsgemisch wird auf 800 ml Eiswasser gegossen und mit 400 ml Aether extrahiert. Der Extrakt wird mit Wasser gewaschen, über Magnesiumsulfat getrocknet und eingedampft. Das durch Säulenchromatographie (Silicagel; Chloroform/Methanol 30:1) gereinigte Produkt wird in Aethylacetat gelöst, mit einem Ueberschuss von Chlorwasserstoff in Aether versetzt und nochmals eingedampft. Der amorphe Rückstand wird in Aethylacetat gelöst, mit Aether versetzt, angeimpft und über Nacht bei Raumtemperatur gerührt. Das Kristallisat wird abfiltriert, mit Aether gewaschen und getrocknet. Man erhält 2-[2-[(3,4-Dimethoxyphenäthyl)methylamino]äthyl -6-fluor-1,2,3,4-tetrahydro -1α-isopropyl -2α-naphthalinol-hydrochlorid, Smp. 190-192°.

In analoger Weise wie oben beschrieben erhält man durch Kondensation der substituierten 2-(1,2,3,4-Tetrahydro-1α-alkyl-2-hydroxy-2β-naphthyl)äthyl -p-toluolsulfonate mit den entsprechenden N-Methyl-arylalkylaminen die folgenden Verbindungen:

- 6-Chlor-2-[2-[(3,4-dimethoxyphenäthyl)methylamino]-äthyl]-1,2,3,4-tetrahydro-1α-isopropyl-2α-naphthalinol-hydrochlorid, Smp. 195-198°,
- 2-[2-[(3,4-Dimethoxyphenäthyl)methylamino]äthyl]-1,2,3,4-tetrahydro-1α-isopropyl-2α-naphthalinol-hydrochlorid, Smp. 175-177°,
- 6,7-Dimethoxy-2-[2-[(3,4-dimethoxyphenäthyl)methylamino]-äthyl]-l,2,3,4-tetrahydro-1α-isopropyl-2α-naphthalinol, Smp. 119-120°,
- 2-[2-[(3,4-Dimethoxyphenäthyl)methylamino]äthyl]-1,2,3,4-tetrahydro-1α-isopropyl-5,6,7-trimethoxy-2α-naphthalinol-hydrochlorid, Smp. 170-172°,
- 6,7-Dimethoxy-2-[2-[[3-(3,4-dimethoxyphenyl)propyl]-methyl-amino]äthyl]-1,2,3,4-tetrahydro-1α-isopropyl-2α-naphthalinol-hydrochlorid, Smp. 110-113°,
- 6-Fluor-1,2,3,4-tetrahydro-1α-isopropyl-2-[2-[(2,4,6-trimethoxyphenäthyl)methylamino]äthyl]-2α-naphthalinol-hydrochlorid, Smp. 146-148°,

- 6-Fluor-1,2,3,4-tetrahydro-1α-isopropyl-2-[2-[(3,4,5-trimethoxyphenäthyl)methylamino]äthyl]-2α-naphthalinol-hydrochlorid, Smp. 173-175°,
- [1S,2S]-2-[2-[(3,4-Dimethoxyphenäthyl)methylamino]äthyl]-6-fluor-1,2,3,4-tetrahydro-1-isopropyl-2-naphthalinol-hydrochlorid, Smp. 164-165°, $[\alpha]_D^{20}$ = +45,3° (c = 1, Methanol),
- 2-[2-[(2,5-Dimethoxyphenäthyl)methylamino]äthyl]-6-fluor-1,2,3,4-tetrahydro-1α-isopropyl-2α-naphthalinol-hydrochlorid, Smp. 164-166°,
- 6-Fluor-1,2,3,4-tetrahydro-1α-isopropyl-2-[2-[(2-(2,2,2-trifluoräthoxy)phenäthyl)methylamino]äthyl]-2α-naphthalinol-hydrochlorid, Smp. 146-148°,
- 6-Fluor-1,2,3,4-tetrahydro-1α-isopropyl-2-[2-[(3-(2,2,2-trifluoräthoxy)phenäthyl)methylamino]äthyl]-2α-naphthalinol-hydrochlorid,
- 6-Fluor-1,2,3,4-tetrahydro-1α-isopropyl-2-[2-[(3-methoxy-4-(methylthio)phenäthyl)methylamino]äthyl]-2α-naphthalinol-hydrochlorid, Smp. 190-192°,
- 6-Fluor-2-[2-[(4-fluorphenäthyl)methylamino]äthyl]-1,2,3,4-tetrahydro-1α-isopropyl-2α-naphthalinol-hydrochlorid, Smp. 148-150°,
- 1α-Butyl-2-[2-[(3,4-dimethoxyphenäthyl)methylamino]-äthyl]-6-fluor-1,2,3,4-tetrahydro-2α-naphthalinol-hydrochlorid, Smp. 123-125°,
- 6-Fluor-1,2,3,4-tetrahydro-1α-isopropyl-2-[2-[(4-methoxyphenäthyl)methylamino]äthyl]-2α-naphthalinol-hydrochlorid, Smp. 123-125°,
- 2-[2-[(4-Butoxyphenäthyl)methylamino]äthyl]-6-fluor-1,2,3,4-tetrahydro-1α-isopropyl-2α-naphthalinol-hydrochlorid, Smp. 169-170° und
- 2-[2-[(4-Decyloxyphenäthyl)methylamino]äthyl]-6-fluor-1,2,3,4-tetrahydro-1α-isopropyl-2α-naphthalinol-hydrochlorid, Smp. 137-139°.

Das in obigem Absatz (A) als Ausgangsmaterial eingesetzte 2-(6-Fluor-1,2,3,4-tetrahydro -2-hydroxy-1α-isopropyl-2β-naphthyl)äthyl-p-toluolsulfonat kann wie folgt hergestellt werden:

(B) 20,0 g (79,3 mMol) 6-Fluor-1,2,3,4-tetrahydro -2-hydroxy-1α-isopropyl-2β-naphthalinäthanol werden in 140 ml Pyridin gelöst und auf 0° gekühlt. Nach Zugabe von 18,2 g (95,5 mMol) p-Toluolsulfonylchlorid wird das Gemisch ohne Kühlung 3 Stunden gerührt und dann auf 1 1 Wasser gegossen. Das Produkt wird mit 600 ml Aether extrahiert, der Aetherextrakt mit 600 ml 3N wässriger Salzsäure, 600 ml gesättig ter wässriger Natriumbicarbonat-Lösung und 600 ml Wasser gewaschen. Nach dem Trocknen über Magnesiumsulfat wird der Aether unter vermindertem Druck abgedampft, und das verbleibende Oel aus 300 ml Cyclohexan umkristallisiert. Man erhält 2-(6-Fluor-1,2,3,4-tetrahydro -2-hydroxy-1α-isopropyl-2β-naphthyl)-äthyl-p-toluolsulfonat, Smp. 91-92°.

In analoger Weise wie oben beschrieben erhält man durch Umsetzen der entsprechenden l,2,3,4-Tetrahydro-2-hydroxy -1α-alkyl-2β-naphthalinäthanole mit p-Toluolsulfonylchlorid die folgenden Verbindungen:

- 2-(6-Chlor-1,2,3,4-tetrahydro-2-hydroxy-1α-isopropyl-2β-naphthyl)äthyl-p-toluolsulfonat,
- 2-(1,2,3,4-Tetrahydro-2-hydroxy-1α-isopropyl-2β-naphthyl)äthyl-p-toluolsulfonat,
- 2-(6,7-Dimethoxy-1,2,3,4-tetrahydro-2-hydroxy-1α-isopropyl-2β-naphthyl)äthyl-p-toluolsulfonat, Smp. 74-75°,
- 2-(1,2,3,4-Tetrahydro-2-hydroxy-1α-isopropyl-5,6,7-trimethoxy-2β-naphthyl)äthyl-p-toluolsulfonat
- 2-(1α-Butyl-6-fluor-1,2,3,4-tetrahydro-2-hydroxy-2β-naphthyl)äthyl-p-toluolsulfonat und
- [1S,2S]-2-(6-Fluor-1,2,3,4-tetrahydro-2-hydroxy-1-isopropyl-2-naphthyl)äthyl-p-toluolsulfonat, Smp. 66-67°.

Die Derivate, die bei Raumtemperatur nicht kristallisieren, werden mit Hilfe spektroskopischer Methoden charakterisiert.

Das in obigem Absatz (B) als Ausgangsmaterial eingesetzte 6-Fluor-1,2,3,4-tetrahydro -2-hydroxy-1α-isopropyl-2β-naphthalinäthanol kann wie folgt hergestellt werden:

(C) Ein Zehntel einer Lösung von 57 g (0,276 Mol) 6-Fluor-3,4-dihydro-1-isopropyl-2(1H)-naphthalinon und 59,5 g (0,305 Mol) Bromessigsäure-tert.-butylester in 330 ml Tetrahydrofuran wird zu 10,6 g (0,436 Mol) aktiviertem Magnesium gegeben. Nachdem die durch Erhitzen verursachte, visuell er kennbare Reaktion einsetzt, wird der Rest der Lösung unter Rückfluss innert 30 Minuten zugetropft. Nach beendigter Zugabe wird das Reaktionsgemisch weitere 1 1/2 Stunden zum Rückfluss erhitzt. Das abgekühlte Gemisch wird langsam mit einer Lösung von 40 g Ammoniumchlorid in 400 ml Wasser versetzt und dann zwischen 600 ml Wasser und 1,5 l Methylenchlorid verteilt. Die organische Phase wird mit Wasser gewaschen, über Magnesiumsulfat getrocknet und eingeengt. Nach säulenchromatographischer Reinigung (Silicagel,Hexan/Aethylacetat 5:1) erhält man tert.-Butyl 6-fluor-1,2,3,4-tetrahydro-2-hydroxy-1α-isopropyl -2β-naphthalinacetat. Das ölige Produkt wird in 415 ml Tetrahydrofuran gelöst und innert 30 Minuten zu

einer gerührten Suspension von 13,44 g (0,354 Mol) Lithiumaluminiumhydrid in 400 ml Tetrahydrofuran getropft, wobei die Reaktionstemperatur auf 60° steigt. Das Gemisch wird weitere 30 Minuten gerührt und dann auf 10° abgekühlt. Vorsichtig werden nacheinander 22 ml Wasser, 22 ml 28%ige wässrige Natronlauge und 66 ml Wasser zugegeben. Die erhaltene Suspension wird filtriert, der Filterkuchen mit Tetrahydrofuran gewaschen, und das Filtrat eingedampft. Der kristalline Rückstand wird aus 120 ml Cyclohexan umkristallisiert. Man erhält 6-Fluor-1,2,3,4-tetrahydro -2-hydroXY-1α-isopropyl-2β-naphthalinäthanol, Smp. 97-98°.

In analoger Weise wie oben beschrieben erhält man durch Umsetzen der entsprechenden 3`4-Dihydro-1-alkyl-2(1H)-naphthalinone mit Magnesium/Bromessigsäure-tert.-butylester und anschliessender Reduktion die folgenden Verbindungen:

- 6-Chlor-1,2,3,4-tetrahydro-2-hydroxy-1α-isopropyl-2β-naphthalinäthanol, Smp. 99-100°,
- 6,7-Dimethoxy-1,2,3,4-tetrahydro-2-hydroxy-1α-isopropyl-2β-naphthalinäthanol, Sdp. 170°/13 Pa,
- 1,2,3,4-Tetrahydro-2-hydroxy-1α-isopropyl-5,6,7-trimethoxy-2β-naphthalinäthanol, Smp. 96-97°,
- 1α-Butyl-6-fluor-1,2,3,4-tetrahydro-2-hydroxy-2β-naphthalinäthanol und
- [1S,2S]-6-Fluor-1,2,3,4-tetrahydro-2-hydroxy-1-isopropyl-2-naphthalinäthanol, Smp. 78-80°, $[\alpha]_D^{20}$ = + 83° (c = 1, Methanol).

(D) 1,2,3,4-Tetrahydro -2-hydroxy-1α-isopropyl-2β-naphthalinäthanol lässt sich wie folgt herstellen:

10,0 g (37,2 mMol) 6-Chlor-1,2,3,4-tetrahydro -2-hydroxy-1α-isopropyl-2β-naphthalinäthanol und 4`5 g Triäthylamin in 150 ml Methanol werden in Gegenwart von 1,9 g 5%igem Palladium auf Kohle bei Raumtemperatur und Atmosphärendruck hydriert. Die filtrierte Reaktionslösung wird eingedampft, und der Rückstand durch Säulenchromatographie (Silicagel, Hexan/Aethylacetat 1:11) gereinigt. Nach Umkristallisation aus Aethylacetat/Hexan erhält man 1,2,3,4-Tetrahydro -2-hydroxy-1α-isopropyl-2β-naphthalinäthanol, Smp. 103-104°.

Das in obigem Absatz (C) als Ausgangsmaterial eingesetzte 6-Fluor-3,4-dihydro -1-isopropyl-2(1H)-naphthalinon kann wie folgt hergestellt werden:

50 g (0,255 Mol) 2-(p-Fluorphenyl)-3-methylbuttersäure werden in 200 ml Benzol gelöst und mit 54,2 g (0,456 Mol) Thionylchlorid versetzt. Das Gemisch wird bei 60° 45 Minuten gerührt und anschliessend 2 Stunden zum Rückfluss erhitzt. Nach dem Abdampfen des Lösungsmittels wird der Rückstand destilliert, wobei man 2-(4-Fluorphenyl)-3-methylbutyryl-chlorid, Sdp. 70°/93 Pa, erhält.

79 g (0,368 Mol) dieses Säurechlorids werden in 2,7 1 Methylenchlorid gelöst und auf -10° abgekühlt. Ein Strom von Aethylengas wird während 1 Stunde in die Lösung eingeleitet. Dann werden 122,4 g (0,918 Mol) wasserfreies Aluminiumchlorid zugegeben und weitere l5 Minuten Aethylengas eingeleitet. Das Gemisch wird 1 Stunde bei 0° gerührt und dann langsam mit 900 ml Wasser versetzt, wobei die Temperatur unter 25° gehalten wird. Die organische Phase wird mit 1 1 3N wässriger Salzsäure und 1 1 gesättigter wässriger Natriumbicarbonatlösung gewaschen, über Magnesiumsulfat getrocknet und eingedampft. Das Rohprodukt wird durch fraktionierte Destillation im Vakuum gereinigt. Man erhält 6-Fluor-3,4-dihydro-1-isopropyl-2(1H)-naphthalinon, Sdp. 100-105°/53 Pa.

In analoger Weise wie oben beschrieben erhält man durch Umsetzen der entsprechenden Säurechloride mit Aethylen und Aluminiumchlorid die folgenden Verbindungen:

- 6-Chlor-3,4-dihydro-1-isopropyl-2(1H)-naphthalinon, Sdp. 100°/40 Pa,
- 3,4-Dihydro-6,7-dimethoxy-1-isopropyl-2(1H)-naphthalinon, Smp. 78-79°,
- 3,4-Dihydro-1-isopropyl-5,6,7-trimethoxy-2(1H)-naphthalinon, Sdp. 140°/13 Pa,
- 1-Butyl-6-fluor-3,4-dihydro-2(1H)-naphthalinon, Sdp. 110-112°/67 Pa und
- [1S]-6-Fluor-3,4-dihydro-1-isopropyl-2(1H)-naphthalinon, $[\alpha]_D^{20}$ = -188° (c = 1, Hexan).

## Beispiel 2

Das in Beispiel 1(C) genannte 6,7-Dimethoxy-1,2,3,4-tetrahydro-2-hydroxy-1α-isopropyl -2β-naphthalinäthanol kann auch auf folgendem Weg gewonnen werden:

22,0 g (0,106 Mol) 3,4-Dihydro-6,7-dimethoxy-2(1H)-naphthalinon werden in 300 ml Dimethylformamid gelöst und mit 13 g (0,116 Mol) Kalium-tert.-butylat versetzt. Die Lösung wird bei 18° 30 Minuten gerührt. Nach Zugabe von 19,7 g (0,116 Mol) Isopropyljodid wird das Gemisch weitere 30 Minuten gerührt, wobei die Temperatur unterhalb 30° gehalten wird, und dann auf Wasser gegossen. Das Produkt wird mit Aether extrahiert, der Extrakt mit Wasser gewaschen, über Magnesiumsulfat getrocknet und eingedampft. Das zurückbleibende Oel wird durch Säulenchromatographie (Silicagel, Hexan/Aethylacetat 6:1) gereinigt. Man erhält 3,4-Dihydro-6.7-dimethoxy -1-isopropyl-2(1H)-naphthalinon, Smp. 77-79°.

Ein Drittel einer Lösung von 28,0 g (0.113 Mol) 3,4-Dihydro-6,7-dimethoxy -1-isopropyl-2(1H)-naphthalinon und 20.9 g (0,125 Mol) Bromessigsäureäthylester in 180 ml Toluol/Benzol 1:1 wird zu 8.3 g (0.127 Mol) aktiviertem Zinkstaub gegeben.

Nach einigen Minuten Erhitzen zum Rückfluss setzt eine exotherme Reaktion ein. Der Rest der Lösung wird dann innerhalb von 10 Minuten zugetropft. Nach beendigter Zugabe wird das Reaktionsgemisch 1 Stunde zum Rückfluss erhitzt, danach abgekühlt und zwischen 300 ml Benzol und 250 ml 3N wässriger Schwefelsäure verteilt. Die organische Phase wird mit Wasser und gesättigter Natriumbicarbonatlösung gewaschen und eingedampft. Der ölige Rückstand wird in 300 ml Methanol gelöst, mit 20 g Natriumhydroxid in 150 ml Wasser versetzt, und das Gemisch 1 Stunde zum Rückfluss erhitzt. Man verdünnt dann die Lösung mit 1.6 l Wasser und extrahiert die neutralen Bestandteile mit 900 ml Aether. Die wässrige Phase wird mit Salzsäure sauer gestellt, und das Produkt in 1200 ml Aether extrahiert. Der Extrakt wird über Magnesiumsulfat getrocknet und eingedampft. Umkristallisation aus Toluol liefert 6,7-Dimethoxy-1,2,3,4-tetrahydro -2-hydroxy-1α-isopropyl-2β-naphthalinessigsäure, Smp. 99-101°.

16.9 g (55 mMol) 6,7-Dimethoxy-1,2,3,4-tetrahydro -2-hydroxy-1α-isopropyl-2β-naphthalinessigsäure werden in 175 ml Tetrahydrofuran gelöst. 17.6 ml (0.176 Mol) einer 10M Lösung des Boran-Methylsulfid-Komplexes in Tetrahydrofuran werden bei 20° innert 30 Minuten zugetropft. Das Reaktionsgemisch wird bei Raumtemperatur 48 Stunden gerührt. Nach Zugabe von 50 ml Methanol wird die Lösung eingedampft. Der Rückstand wird in 300 ml Methanol aufgenommen und 3 Stunden zum Rückfluss erhitzt. Das nach dem Abdampfen des Methanols verbleibende Oel wird durch Säulenchromatographie (Silicagel, Chloroform/Methanol 40:1) gereinigt und anschliessend unter vermindertem Druck destilliert. Man erhält 6,7-Dimethoxy-1,2,3,4-tetrahydro -2-hydroxy-1α-isopropyl-2β-naphthalinäthanol, Sdp. 180°/27 Pa.

## Beispiel 3

19,8 g (46.1 mMol) 2-[2-[(3,4-Dimethoxyphenäthyl)methylamino]äthyl] -6-fluor-1,2,3,4-tetrahydro-1α-isopropyl -2α-naphthalinol werden in 140 ml Chloroform gelöst und auf 0° gekühlt. 10,0 g (92,2 mMol) Methoxyacetylchlorid werden zugegeben, und das Gemisch bei 0° 3 Stunden gerührt. Die Reaktionslösung wird auf 500 ml eiskalte 1N wässrige Natronlauge gegossen, die organische Phase mit Wasser gewaschen, über Magnesiumsulfat getrocknet und eingedampft. Der Rückstand wird in Aethylacetat gelöst, mit einem Ueberschuss von Chlorwasserstoff in Aether versetzt und nochmals eingedampft. Der amorphe Rückstand wird in Aethylacetat gelöst, mit Aether versetzt, angeimpft und über Nacht bei Raumtemperatur gerührt. Das Kristallisat wird abfiltriert, mit Aether gewaschen und getrocknet. Man erhält 2-[2-[(3,4-Dimethoxyphenäthyl)methylamino]äthyl] -6-fluor-1,2,3,4-tetrahydro-1α-isopropyl -2α-naphthylmethoxyacetat-hydrochlorid, Smp. 150-152°.

In analoger Weise wie oben beschrieben erhält man durch Umsetzen der entsprechenden 1,2,3,4-Tetrahydro-2-naphthalinol-Derivate mit Carbonsäurechloriden die folgenden Verbindungen:

- 6-Chlor-2-[2-[(3,4-dimethoxyphenäthyl)methylamino]-äthyl]-1,2,3,4-tetrahydro-1α-isopropyl-2α-naphthylmethoxyacetat-hydrochlorid, Smp. 177-180°.
- 2-[2-[(3,4-Dimethoxyphenäthyl)methylamino]äthyl]-1,2,3,4-tetrahydro-1α-isopropyl-2α-naphthylmethoxyacetat-hydrochlorid, Smp. 176-178°,
- 6,7-Dimethoxy-2-[2-[(3,4-dimethoxyphenäthyl)methylamino]äthyl]-1,2,3,4-tetrahydro-1α-isopropyl-2α-naphthylmethoxyacetat-hydrochlorid, Smp. 183-186°,
- 2-[2-[(3,4-Dimethoxyphenäthyl)methylamino]äthyl]-1,2,3,4-tetrahydro-1α-isopropyl-5,6,7-trimethoxy-2α-naphthylmethoxyacetat-hydrochlorid, Smp. 162-164°,
- 6,7-Dimethoxy-2-[2-[(3-/3,4-dimethoxyphenyl/propyl)-methylamino]äthyl]-1,2,3,4-tetrahydro-1α-isopropyl-2α-naphthylmethoxyacetat-hydrochlorid, Smp. 148-150°,
- 6-Fluor-1,2,3,4-tetrahydro-1α-isopropyl-2-[2-[(2,4,6-trimethoxyphenäthyl)methylamino]äthyl]-2α-naphthylmethoxyacetat-hydrochlorid, Smp. 138-140°,
- 6-Fluor-1,2,3,4-tetrahydro-1α-isopropyl-2-[2-[(3,4,5-trimethoxyphenäthyl)methylamino]äthyl]-2α-naphthylmethoxyacetat-hydrochlorid, Smp. 157-159°,
- 2-[2-[(2,5-Dimethoxyphenäthyl)methylamino]äthyl]-6-fluor-1,2,3,4-tetrahydro-1α-isopropyl-2α-naphthylmethoxyacetat-hydrochlorid, Smp. 161-163°,
- 6-Fluor-1,2,3,4-tetrahydro-1α-isopropyl-2-[2-[(2-/2,2,2-trifluoräthoxy/phenäthyl)methylamino]äthyl]-2α-naphthylmethoxyacetat-hydrochlorid, Smp. 169-171°,
- 6-Fluor-1,2,3,4-tetrahydro-1α-isopropyl-2-[2-[(3-(2,2,2-trifluoräthoxy)phenäthyl)methylamino]äthyl]-2α-naphthylmethoxyacetat-hydrochlorid, Smp. 167-169°,
- 6-Fluor-1,2,3,4-tetrahydro-1α-isopropyl-2-[2-[(3-methoxy-4-(methylthio)phenäthyl)methylamino]äthyl]-2α-naphthylmethoxyacetat-hydrochlorid, Smp. 157-159°,

- 6-Fluor-2-[2-[(4-fluorphenäthyl)methylamino]äthyl]-1,2,3,4-tetrahydro-1α-isopropyl-2α-naphthylmethoxyacetat-hydrochlorid, Smp. 193-195°,
- 1α-Butyl-2-[2-[(3,4-dimethoxyphenäthyl)methylamino]-äthyl]-6-fluor-1,2,3,4-tetrahydro-2α-naphthylmethoxyacetat-hydrochlorid, Smp. 108-110°,
- 6-Fluor-1,2,3-4-tetrahydro-1α-isopropyl-2-[2-[(4-methoxyphenäthyl)methylamino]äthyl]-2α-naphthylmethoxyacetat-hydrochlorid, Smp. 204-206°,
- 2-[2-[(4-Butoxyphenäthyl)methylamino]äthyl]-6-fluor-1,2,3,4-tetrahydro-1α-isopropyl-2α-naphthylmethoxyacetat-hydrochlorid, Smp. 134-135°,
- 2-[2-[(4-Decyloxyphenäthyl)methylamino]äthyl]-6-fluor-1,2,3,4-tetrahydro-1α-isopropyl-2α-naphthylmethoxyacetat-hydrochlorid, Smp. 105-106°,
- 2-[2-[(3,4-Dimethoxyphenäthyl)methylamino]äthyl]-6-fluor-1,2,3,4-tetrahydro-1α-isopropyl-2α-naphthyläthylcarbonat-hydrochlorid, Smp. 94-97°,
- 2-[2-[(3,4-Dimethoxyphenäthyl)methylamino]äthyl]-6-fluor-1,2,3,4-tetrahydro-1α-isopropyl-2α-naphthyl-(2-methoxyäthyl)carbonat-hydrochlorid, Smp. 113-115°,
- 2-[2-[(3,4-Dimethoxyphenäthyl)methylamino]äthyl]-6-fluor-1,2,3,4-tetrahydro-1α-isopropyl-2α-naphthyl-(methylthio)acetat-hydrochlorid, Smp. 110-112°,
- 2-[2-[(3,4-Dimethoxyphenäthyl)methylamino]äthyl]-6-fluor-1,2,3,4-tetrahydro-1α-isopropyl-2α-naphthyl-(4-methoxyphenyl)acetat-hydrochlorid, Smp. 129-131°,
- 6,7-Dimethoxy-2-[2-[(3,4-dimethoxyphenäthyl)methylamino]-äthyl]-1,2,3,4-tetrahydro-1α-isopropyl-2α-naphthylbutyrat, Smp. 104-106° und
- [1S,2S]-2-[2-[(3,4-Dimethoxyphenäthyl)methylamino]äthyl]-6-fluor-1,2,3,4-tetrahydro-1-isopropyl-2-naphthylmethoxyacetat-hydrochlorid, Smp. 175-176°, $[\alpha]_D^{20}$ = + 35,2° (c = 1, Methanol).

<u>Beispiel 4</u>

3,97 g (8,85 mMol) 2-(6,7-Dimethoxy-1,2,3,4-tetrahydro -2-hydroxy-1α-isopropyl-2β-naphthyl)äthyl-p-toluolsulfonat und 3,96 g (17,7 mMol) 4-[(3,4-Dimethoxyphenyl)butyl]methylamin werden in 7 ml Dimethylsulfoxid gelöst und 1 Stunde bei 80° gerührt. Das Reaktionsgemisch wird zwischen Aethylacetat und Wasser verteilt, und die organische Phase mit Wasser gewaschen, über Magnesiumsulfat getrocknet und eingeengt. Das durch Säulenchromatographie (Silicagel, Chloroform/Methanol 25:1) gereinigte Produkt wird, wie oben beschrieben, in das Hydrochlorid überführt. Man erhält 6,7-Dimethoxy -2-[2-[[4--(3,4-dimethoxyphenyl)butyl]methylamino]äthyl] -1,2,3,4-tetrahydro-1α-isopropyl -2α-naphthalinol-hydrochlorid, Smp. 137-139°.

In analoger Weise wie oben beschrieben erhält man durch Kondensation der in Beispiel 1 genannten, substituierten 2-(1,2,3,4-Tetrahydro -2-hydroxy-1α-isopropyl-2β-naphthyl)äthyl -p-toluolsulfonate mit den entsprechenden N-Methyl-arylalkylaminen folgende Verbindungen:

- 6-Fluor-1,2,3,4-tetrahydro-1α-isopropyl-2-[2-[(phenäthyl)methylamino]iäthyl]-2α-naphthalinol-hydrochlorid, Smp. 176-177°,
- 6,7-Dimethoxy-1,2,3,4-tetrahydro-1α-isopropyl-2-[2-[[4-methoxy-3-(3-methoxypropoxy)phenäthyl]-methylamino]-äthyl]-2α-naphthalinol und
- 6,7-Dimethoxy-1,2,3,4-tetrahydro-1α-isopropyl-2-[2-[[3-methoxy-4-(3-methoxypropoxy)phenäthyl]-methylamino]-äthyl]-2α-naphthalinol.

<u>Beispiel 5</u>

Eine Lösung von 2,3 g (4,6 mMol) 6,7-Dimethoxy -2-[2-[[4-(3,4-dimethoxyphenyl)butyl]methylamino]-äthyl] -1,2,3,4-tetrahydro-1α-isopropyl-2α-naphthalinol in 20 ml Essigsäureanhydrid wird bei Raumtemperatur 16 Stunden gerührt. Das Reaktionsgemisch wird zu 200 ml Eiswasser gegeben, mit 28%iger wässriger Natronlauge alkalisch gestellt, und das Produkt mit Aethylacetat extrahiert. Der Extrakt wird mit Wasser gewaschen, über Magnesiumsulfat getrocknet und eingedampft. Der Rückstand wird in Aethylacetat gelöst, mit einem Ueberschuss von Chlorwasserstoff in Aether versetzt und nochmals eingedampft. Der amorphe Rückstand wird in Aethylacetat gelöst, mit Aether versetzt und 2 Stunden gerührt. Das gebildete Kristallisat wird abfiltriert, mit Aether gewaschen und getrocknet. Man erhält 6,7-Dimethoxy -2-[2-[[4-(3,4-dimethoxyphenyl)butyl]methylamino]äthyl] -1,2,3,4-tetrahydro-1α-isopropyl -2α-naphthylacetat-hydrochlorid, Smp. 143-145°.

In analoger Weise wie oben beschrieben erhält man durch Umsetzen der entsprechenden 1,2,3,4-Tetrahydro-2-naphthalinol-Derivate mit Essigsäureanhydrid die folgenden Verbindungen:

- 6-Chlor-2-[2-[(3,4-dimethoxyphenäthyl)methylamino]-äthyl]-1,2,3,4-tetrahydro-1α-isopropyl-2α-naphthylacetat-hydrochlorid, Smp. 172-174°,
- 2-[2-[(3,4-Dimethoxyphenäthyl)methylamino]äthyl]-1,2,3,4-tetrahydro-1α-isopropyl-2α-naphthylacetat-hydrochlorid, Smp. 184-185°,
- 6,7-Dimethoxy-2-[2-[(3,4-dimethoxyphenäthyl)methylamino]-äthyl]-1,2,3,4-tetrahydro-1α-isopropyl-2α-naphthylacetat-hydrochlorid, Smp. 194-196°,
- 2-[2-[(3,4-Dimethoxyphenäthyl)methylamino]äthyl]-1,2,3,4-tetrahydro-1α-isopropyl-5,6,7-trimethoxy-2α-naphthylacetat-hydrochlorid (amorph),
- 6,7-Dimethoxy-2-[2-[[3-(3,4-dimethoxyphenyl)propyl]-methylamino]äthyl]-1,2,3,4-tetrahydro-1α-isopropyl-2α-naphthylacetat-hydrochlorid, Smp. 151-153°,
- 6-Fluor-1,2,3,4-tetrahydro-1α-isopropyl-2-[2-[(2,4,6-trimethoxyphenäthyl)methylamino]äthyl]-2α-naphthylacetat-hydrochlorid, Smp. 159-161°,
- 6-Fluor-1,2,3,4-tetrahydro-1α-isopropyl-2-[2-[(2-(2,2,2-trifluoräthoxy)phenäthyl)methylamino]äthyl]-2α-naphthylacetat-hydrochlorid, Smp. 152-154°,
- 6-Fluor-1,2,3,4-tetrahydro-1α-isopropyl-2-[2-[(3-(2,2,2-trifluoräthoxy)phenäthyl)methylamino]äthyl]-2α-naphthylacetat-hydrochlorid, Smp. 163-165°,
- 1α-Butyl-2-[2-[(3,4-dimethoxyphenäthyl)methylamino]-äthyl]-6-fluor-1,2,3,4-tetrahydro-2α-naphthylacetat-hydrochlorid, Smp. 117-119°,
- 6-Fluor-1,2,3,4-tetrahydro-1α-isopropyl-2-[2-[(phenäthyl)methylamino]äthyl]-2α-naphthylacetat-hydrochlorid, Smp. 225-227°,
- 2-[2-[(3,4-Dimethoxyphenäthyl)methylamino]äthyl]-6-fluor-1,2,3,4-tetrahydro-1α-isopropyl-2α-naphthylacetat-hydrochlorid, Smp. 169-171°.
- 6,7-Dimethoxy-1,2,3,4-tetrahydro-1α-isopropyl-2-[2-[[4-methoxy-3-(3-methoxypropoxy)phenäthyl]-methylamino]-äthyl]-2α-naphthylacetat-hydrochlorid, Smp. 143-145° und
- 6,7-Dimethoxy-1,2,3,4-tetrahydro-1α-isopropyl-2-[2-[[3-methoxy-4-(3-methoxypropoxy)phenäthyl]-methylamino]-äthyl]-2α-naphthylacetat-hydrochlorid, Smp. 136-138°.

Beispiel 6

(A) 17,0 g (41.8 mMol) 2-(5,8-Dimethoxy-1,2,3,4-tetrahydro -2-hydroxy-2-naphthyl)äthyl-p-toluolsulfonat und 16,3 g (83,6 mMol) N-Methylhomoveratrylamin werden 30 Minuten auf 100° erhitzt. Das Reaktionsgemisch wird zwischen Aethylacetat und Wasser verteilt, die organische Phase über Magnesiumsulfat getrocknet und eingedampft. Nach säulenchromatographischer Reinigung (Silicagel, Chloroform/Methanol 40:1) erhält man 5,8-Dimethoxy -2-[2-[(3,4-dimethoxyphenäthyl)methylamino]äthyl] -1,2,3,4-tetrahydro-2-naphthalinol, das in das Hydrochlorid überführt wird, Smp. 156-158°.

In analoger Weise erhält man aus 2-(6,7-Dimethoxy--1,2,3,4-tetrahydro -2-hydroxy-2-naphthyl)äthyl-p-toluolsulfonat das 6,7-Dimethoxy -2-[2-[(3,4-dimethoxyphenäthyl)-methylamino]äthyl] -1,2,3,4-tetrahydro-2-naphthalinol-hydrochlorid, Smp. 175-177°.

Das in obigem Absatz (A) als Ausgangsmaterial verwendete 2-(5,8-Dimethoxy -1,2,3,4-tetrahydro-2-hydroxy -2-naphthyl)-äthyl-p-toluolsulfonat kann wie folgt hergestellt werden: (B) 11,5 g (45,6 mMol) 5`8-Dimethoxy-1,2,3,4-tetrahydro -2-hydroxy-2-naphthalinäthanol werden in 70 ml Pyridin gelöst und mit 10,5 g (55 mMol) p-Toluolsulfonylchlorid versetzt. Das Gemisch wird bei 0° 16 Stunden gerührt und dann auf 500 ml Wasser gegossen. Das Produkt wird in 500 ml Aether extrahiert, der Extrakt mit 0,5N wässriger Salzsäure und gesättigter wässriger Natriumbicarbonatlösung gewaschen und über Magnesiumsulfat getrocknet. Nach dem Eindampfen verbleiben 2-(5,8-Dimethoxy -1,2,3,4-tetrahydro-2-hydroxy-2-naphthyl)äthyl -p-toluolsulfonat.

In analoger Weise erhält man durch selektive Tosylierung von 6,7-Dimethoxy-1,2,3,4-tetrahydro -2-hydroxy-2-naphthalinäthanol das 2-(6,7-Dimethoxy -1,2,3,4-tetrahydro-2-hydroxy -2-naphthyl)äthyl-p-toluolsulfonat.

Das in obigem Absatz (B) als Ausgangsmaterial verwendete 5,8-Dimethoxy-1,2,3,4-tetrahydro -2-hydroxy-2-naphthalinäthanol kann wie folgt hergestellt werden:

(C) 15,5 g (58,2 mMol) 5,8-Dimethoxy-1,2,3,4-tetrahydro -2-hydroxy-2-naphthalinessigsäure in 150 ml Tetrahydrofuran werden innert 15 Minuten zu 3,32 g (87,5 mMol) Lithiumaluminiumhydrid in 150 ml Tetrahydrofuran getropft. Das Reaktionsgemisch wird 1 Stunde zum Rückfluss erhitzt und dann abgekühlt. Nach tropfenweiser Zugabe von 15 ml Aethylacetat und 20 ml Wasser wird die Suspension filtriert, und das Filtrat eingeengt. Das Rohprodukt wird durch Säulenchromatographie (Silicagel, Chloroform/Methanol 30:1)

gereinigt. Man erhält 5,8-Dimethoxy-1,2,3,4-tetrahydro -2-hydroxy-2-naphthalinäthanol, Smp. 121-122°.

In analoger Weise erhält man durch Reduktion von 6,7-Dimethoxy-l,2,3,4-tetrahydro -2-hydroxy-2-naphthalinessigsäure das 6,7-Dimethoxy-1,2,3,4-tetrahydro -2-hydroxy-2-naphthalinäthanol, Smp. 91-93°.

(D) Die in obigem Absatz (C) als Ausgangsmaterial verwendete 5,8-Dimethoxy-1,2,3,4-tetrahydro -2-hydroxy-2-naphthalinessigsäure kann wie folgt hergestellt werden:

Ein Fünftel einer Lösung von 20,6 g (0,1 Mol) 3,4-Dihydro -5,8-dimethoxy-2(1H)-naphthalinon und 18,4 g (0.11 Mol) Bromessigsäureäthylester in 100 ml Toluol/Benzol (1:1) wird zu 7,4 g (0,113 Mol) aktiviertem Zinkstaub gegeben. Nach einigen Minuten Erhitzen zum Rückfluss setzt eine exotherme Reaktion ein. Dann wird der Rest der Lösung innert 10 Minuten zugetropft. Das Reaktionsgemisch wird dann 1 1/2 Stunden zum Rückfluss erhitzt, abgekühlt und zwischen 300 ml Benzol und 300 ml 3N wässriger Schwefelsäure verteilt. Die organische Phase wird mit Wasser und gesättigter wässriger Natriumbicarbonatlösung gewaschen und eingedampft. Der Rückstand wird in 200 ml Methanol gelöst, mit einer Lösung von 10 g Natriumhydroxid in 100 ml Wasser versetzt, und das Gemisch 3 Stunden zum Rückfluss erhitzt. Die Lösung wird auf das halbe Volumen eingedampft, mit 500 ml Wasser versetzt, und die neutralen Bestandteile mit 500 ml Chloroform extrahiert. Die wässrige Phase wird mit Schwefelsäure sauer gestellt, und das Produkt in 900 ml Aether extrahiert. Der Extrakt wird über Magnesiumsulfat getrocknet und eingedampft. Umkristallisation des Rückstandes liefert 5,8-Dimethoxy-1,2,3,4-tetrahydro -2-hydroxy-2-naphthalinessigsäure, Smp. 112-114°.

In analoger Weise kann durch Umsetzen von 3,4-Dihydro -6,7-dimethoxy-2(1H)-naphthalinon mit Zink/Bromessigsäureäthylester und anschliessender Verseifung die 6,7-Dimethoxy--1,2,3,4-tetrahydro -2-hydroxy-2-naphthalinessigsäure erhalten werden, Smp. 100-102°.

## Beispiel 7

(A) 4,3 g (10 mMol) 5,8-Dimethoxy -2-[2-[(3,4-dimethoxyphenäthyl)methylamino]äthyl] -1,2,3,4-tetrahydro-2-naphthalinol werden in 15 ml Essigsäureanhydrid gelöst und bei Raumtemperatur 18 Stunden gerührt. Das Reaktionsgemisch wird auf Wasser gegossen, mit Natronlauge alkalisch gestellt, und das Produkt in Chloroform extrahiert. Das Lösungsmittel wird eingedampft, und der Rückstand in Aethylacetat gelöst, mit einem Ueberschuss von Chlorwasserstoff in Aether versetzt und erneut eingedampft. Kristallisation des amorphen Rückstandes aus Aethylacetat liefert 5,8-Dimethoxy -2-[2-[(3,4-dimethoxyphenäthyl)-methylamino]äthyl] -1,2,3,4-tetrahydro-2-naphthylacetat-hydrochlorid, Smp. 115-117°.

In analoger Weise erhält man durch Acetylieren von 6,7-Dimethoxy -2-[2-[(3,4-dimethoxyphenäthyl)-methylamino]-äthyl] -1,2,3,4-tetrahydro-2-naphthalinol das 6,7-Dimethoxy -2-[2-[(3,4-dimethoxyphenäthyl)-methylamino]äthyl] -1,2,3,4-tetrahydro-2-naphthylacetat-hydrochlorid, Smp. 162-164°.

## Beispiel 8

Ein Gemisch von 2,0 g (6,5 mMol) 6,7-Dimethoxy-1,2,3,4-tetrahydro -2-hydroxy-1α-isopropyl-2β-naphthalinpropionaldehyd, 1,51 g (6,5 mMol) N-Methylhomoveratrylamin-hydrochlorid und 0,33 g (3,3 mMol) Triäthylamin in 35 ml Methanol wird mit 0,41 g (6,5 mMol) Natriumcyanoborhydrid versetzt und 2 Stunden bei Raumtemperatur gerührt. Nach Zugabe von 3,5 ml 36%iger Salzsäure wird das Reaktionsgemisch eingedampft, und der Rückstand zwischen 50 ml 3N wässriger Natronlauge und 100 ml Aether verteilt. Die organische Phase wird eingedampft und das Rohprodukt durch Säulenchromatographie (Silicagel, Chloroform/Methanol 20:1) gereinigt. Man erhält 6,7-Dimethoxy -2-[3-[(3,4-dimethoxyphenäthyl)-methylamino]propyl] -1,2,3,4-tetrahydro-1α-isopropyl-2α-naphthalinol, Smp. 97-99°.

Der als Ausgangsmaterial eingesetzte 6,7-Dimethoxy-1,2,3,4-tetrahydro -2-hydroxy-1α-isopropyl-2β-naphtha linpropionaldehyd kann wie folgt hergestellt werden:

2,8 g (9,2 mMol) 6,7-Dimethoxy-1,2,3,4-tetrahydro -2-hydroxy-1α-isopropyl-2β-naphthalinpropionitril werden in 50 ml Methylenchlorid gelöst und auf 0° gekühlt. Nun werden bei dieser Temperatur innert 10 Minuten 10 ml einer 1,2M Lösung von Diisobutylaluminiumhydrid in Toluol (12 mMol) zugetropft, und das Gemisch anschliessend 2 Stunden bei Raumtemperatur gerührt. Man setzt hintereinander 4 ml Methanol, 2,2 ml Wasser und 5 ml Methanol zu, rührt die Suspension für 30 Minuten und filtriert. Das Filtrat wird eingeengt, und das Rohprodukt durch Säulenchromatographie (Silicagel, Hexan/Aethylacetat 1:2) gereinigt. Nach Umkristallisation aus Cyclohexan erhält man 6,7-Dimethoxy-1,2,3,4-tetrahydro -2-hydroxy-1α-isopropy l-2β-naphthalinpropionaldehyd, Smp. 133-135°. Die spektroskopischen Daten (NMR, IR) zeigen, dass die Substanz als Hemiacetal vorliegt.

Das als Ausgangsmaterial eingesetzte, oben genannte 6,7-Dimethoxy-1,2,3,4-tetrahydro -2-hydroxy-1α-isopropyl-2β-naphthalinpropionitril kann wie folgt hergestellt werden:

1,6 g (33 mMol) Natriumcyanid werden in 20 ml Dimethylsulfoxid bei 100° gelöst und auf 40° gekühlt. Man gibt 7,3 g (16,3 mMol) 2-(6,7-Dimethoxy -1,2,3,4-tetrahydro -2-hydroxy-1α-isopropyl-2β-naphthyl)äthyl-p-toluolsulfonat in 10 ml Dimethylsulfoxid zu. Das Gemisch wird bei Raumtemperatur 2 Tage gerührt und anschliessend zwischen 200 ml Wasser und 200 ml Aether verteilt. Die organische Phase wird nochmals mit Wasser gewaschen, eingedampft, und der Rückstand durch Säulenchromatographie (Silicagel, Hexan/Aethylacetat 4:1) gereinigt. Man erhält 6,7-Dimethoxy-1,2,3,4-tetrahydro -2-hydroxy-1α-isopropyl-2β-naphthalinpropionitril, Smp. 82-83°.

Beispiel 9

1,1 g (2,3 mMol) 6,7-Dimethoxy -2-[3-[(3,4-dimethoxyphenäthyl)methylamino]propyl] -1,2,3,4-tetrahydro-1α-isopropyl-2α-naphthalinol werden in 15 ml Essigsäureanhydrid gelöst, und die Lösung 20 Stunden bei 70° gerührt. Das Reaktionsgemisch wird auf Eiswasser gegossen, mit Natronlauge alkalisch gestellt, und das Produkt in Aethylacetat extrahiert. Der eingedampfte Extrakt wird durch Säulenchromatographie (Silicagel, Chloroform/Methanol 25:1) gereinigt, und das Produkt in das Hydrochlorid überführt. Man erhält 6,7-Dimethoxy -2-[3-[(3,4-dimethoxyphenäthyl)methylamino]-propyl] -1,2,3,4-tetrahydro-1α-isopropyl -2α-naphthylacetat-hydrochlorid, Smp. 145-147°.

Beispiel A

## Tabletten

Zusammensetzung:

| | | |
|---|---|---|
| 1) | 2-[2-[(3,4-Dimethoxyphenäthyl)methylamino]äthyl]-6-fluor-1,2,3,4-tetrahydro-1α-isopropyl-2α-naphthylmethoxyacetat-hydrochlorid | 75 mg |
| 2) | Milchzucker pulv. | 135 mg |
| 3) | Maisstärke weiss | 55 mg |
| 4) | Povidone K 30 | 15 mg |
| 5) | Maisstärke weiss | 15 mg |
| 6) | Talk | 3 mg |
| 7) | Magnesiumstearat | 2 mg |
| | Tablettengewicht | 300 mg |

Herstellungsverfahren:

1-3 werden intensiv gemischt. Die Mischung wird danach mit einer wässrigen Lösung von 4 befeuchtet und geknetet, und die resultierende Masse granuliert, getrocknet und ge siebt. Das Granulat wird mit 5-7 gemischt und zu Tabletten geeigneter Grösse verpresst.

Beispiel B

## Tabletten

Zusammensetzung:

| | | | |
|---|---|---|---|
| 1) | 2-[2-[(3,4-Dimethoxyphenäthyl)methyl-amino]äthyl]-6-fluor-1,2,3,4-tetrahydro-1α-isopropyl-2α-naphthyl-methoxyacetat-hydrochlorid | 75 mg | 60 mg |
| 2) | Milchzucker pulv. | 100 mg | 100 mg |
| 3) | Maisstärke | 60 mg | 60 mg |
| 4) | Povidone K 30 | 5 mg | 5 mg |
| 5) | Maisstärke | 15 mg | 15 mg |
| 6) | Natriumcarboxymethylstärke | 5 mg | 5 mg |
| 7) | Talk | 3 mg | 3 mg |
| 8) | Magnesiumstearat | 2 mg | 2 mg |
| | Tablettengewicht | 265 mg | 250 mg |

Herstellungsverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel C

Tabletten

Zusammensetzung:

| | | | |
|---|---|---|---|
| 1) | 2-[2-[(3,4-Dimethoxyphenäthyl)methyl-amino]äthyl]-6-fluor-1,2,3,4-tetrahydro-1α-isopropyl-2α-naphthyl-methoxyacetat-hydrochlorid | 75 mg | 90 mg |
| 2) | Milchzucker pulv. | 46 mg | 46 mg |
| 3) | Cellulose mikrokristallin | 60 mg | 60 mg |
| 4) | Povidone K 30 | 10 mg | 10 mg |
| 5) | Natriumcarboxymethylstärke | 4 mg | 4 mg |
| 6) | Talk | 3 mg | 3 mg |
| 7) | Magnesiumstearat | 2 mg | 2 mg |
| | Tablettengewicht | 200 mg | 215 mg |

Herstellungsverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel D

Kapseln

**Zusammensetzung:**

| | | |
|---|---|---|
| 1) | 2-[2-[(3,4-Dimethoxyphenäthyl)methylamino]äthyl]-6-fluor-1,2,3,4-tetrahydro-1α-isopropyl-2α-naphthylmethoxyacetat-hydrochlorid | 75 mg |
| 2) | Milchzucker krist. | 100 mg |
| 3) | Maisstärke weiss | 20 mg |
| 4) | Talk | 9 mg |
| 5) | Magnesiumstearat | 1 mg |
| | Kapselfüllgewicht | 205 mg |

Herstellungsverfahren:

Der Wirkstoff wird mit dem Milchzucker intensiv gemischt. Dieser Mischung wird danach die Maisstärke, der Talk und das Magnesiumstearat zugemischt, und das Gemisch in Kapseln geeigneter Grösse abgefüllt.

Beispiel E

Kapseln

**Zusammensetzung:**

| | | |
|---|---|---|
| 1) | 2-[2-[(3,4-Dimethoxyphenäthyl)methylamino]äthyl]-6-fluor-1,2,3,4-tetrahydro-1α-isopropyl-2α-naphthylmethoxyacetat-hydrochlorid | 75 mg |
| 2) | Cellulose mikrokristallin | 100 mg |
| 3) | Natriumcarboxymethylstärke | 5 mg |
| 4) | Talk | 9 mg |
| 5) | Magnesiumstearat | 1 mg |
| | Kapselfüllgewicht | 190 mg |

Herstellungsverfahren:

Der Wirkstoff wird mit der Cellulose intensiv gemischt. Dieser Mischung wird danach die Natriumcarboxymethylstärke, den Talk und das Magnesiumstearat zugemischt, und das Gemisch in Kapseln geeigneter Grösse abgefüllt.

Beispiel F

## Injektionslösung

| | | 1 ml |
|---|---|---|
| 2-[2-[(3,4-Dimethoxyphenäthyl)methylamino]äthyl]-6-fluor-1,2,3,4-tetrahydro-1α-isopropyl-2α-naphthylmethoxyacetat-hydrochlorid | | 8 mg |
| Natriumchlorid krist. rein | | 8,5 mg |
| Wasser zu Injektionszwecken | ad | 1 ml |

Beispiel G

Wenn man nach den in den Beispielen A-F beschriebenen Verfahren arbeitet, können aus den folgenden, ebenfalls bevorzugten Verbindungen und ihren pharmazeutisch verwendbaren Salzen Tabletten, Kapseln und Injektionspräparate hergestellt werden:

- [1S,2S]-2-[2-[(3,4-Dimethoxyphenäthyl)methylamino]-äthyl]-6-fluor-1,2,3,4-tetrahydro-1-isopropyl-2-naphthylmethoxyacetat-hydrochlorid.
- 6,7-Dimethoxy-2-[2-[(3,4-dimethoxyphenäthyl)methyl-amino]äthyl]-1,2,3,4-tetrahydro-1α-isopropyl-2α-naphthylmethoxyacetat-hydrochlorid.
- 6,7-Dimethoxy-2-[2-[(3,4-dimethoxyphenäthyl)methyl-amino]äthyl]-1,2,3,4-tetrahydro-1α-isopropyl-2α-naphthylacetat-hydrochlorid.

**Ansprüche**

**1.** Tetrahydronaphthalinderivate der allgemeinen Formel

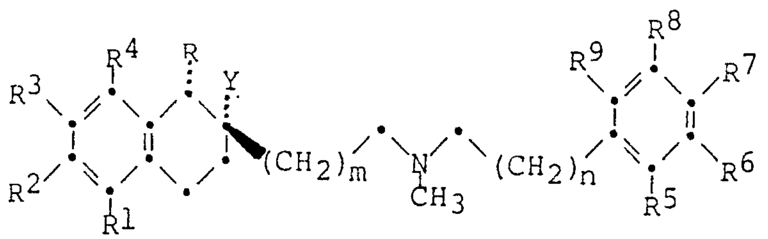

I

worin R Wasserstoff oder nieder-Alkyl, $R^1$, $R^2$, $R^3$ und $R^4$ unabhängig voneinander je Wasserstoff, Halogen oder nieder-Alkoxy oder zwei benachbarte Reste zusammen Methylendioxy oder Aethylendioxy, $R^5$, $R^6$, $R^7$, $R^8$ und $R^9$ unabhängig voneinander je Wasserstoff, Halogen, $C_1$-$C_{10}$-Alkoxy, nieder Alkylthio, nieder--Alkoxy-nieder-alkoxy oder ω,ω,ω-Trifluor-nieder-alkoxy oder zwei benachbarte Reste zusammen Methylendioxy oder Aethylendioxy, Y Hydroxy, nieder-Alkylcarbonyloxy, nieder-Alkoxy-nieder-alkylcarbonyloxy, nieder-Alkoxycarbonyloxy, nieder-Alkoxy-nieder-alkoxycarbonyloxy,

nieder-Alkylthio-nieder-alkylcarbonyloxy oder gegebenenfalls substituiertes Benzylcarbonyloxy, m die Zahl 1 oder 2 und n die Zahl 1, 2 oder 3 bedeuten, in Form von Racematen und optischen Antipoden, sowie pharmazeutisch verwendbare Säureadditionssalze davon.

**2.** Verbindungen gemäss Anspruch 1, worin R nieder-Alkyl bedeutet.

**3.** Verbindungen gemäss Anspruch 2, worin R Isopropyl bedeutet.

**4.** Verbindungen gemäss einem der Ansprüche 1-3, worin Y Hydroxy, nieder-Alkylcarbonyloxy oder nieder-Alkoxy-nieder--alkylcarbonyloxy bedeutet.

**5.** Verbindungen gemäss Anspruch 4, worin Y Hydroxy, Acetyloxy oder Methoxyacetyloxy bedeutet.

**6.** Verbindungen gemäss einem der Ansprüche 1-5, worin m und n je die Zahl 1 bedeuten.

**7.** Verbindungen gemäss einem der Ansprüche 1-6, worin zwei der Substituenten $R^1$, $R^2$, $R^3$ und $R^4$ Wasserstoff und die beiden anderen unabhängig voneinander je Wasserstoff, Halogen oder nieder-Alkoxy bedeuten.

**8.** Verbindungen gemäss Anspruch 7, worin $R^1$ und $R^4$ je Wasserstoff, $R^2$ und $R^3$ je nieder-Alkoxy oder $R^2$ Halogen und $R^3$ Wasserstoff bedeuten.

**9.** Verbindungen gemäss einem der Ansprüche 1-8, worin zwei der Substituenten $R^5$, $R^6$, $R^7$, $R^8$ und $R^9$ Wasserstoff und die drei anderen unabhängig voneinander je Wasserstoff, Halogen, $C_1$-$C_{10}$-Alkoxy, nieder-Alkylthio oder nieder-Alkoxy-nieder-alkoxy bedeuten.

**10.** Verbindungen gemäss Anspruch 9, worin $R^5$ Wasserstoff oder $C_1$-$C_{10}$-Alkoxy, $R^6$ Wasserstoff,$C_1$-$C_{10}$--Alkoxy oder nieder-Alkoxy-nieder-alkoxy, $R^7$ Wasserstoff, Halogen, $C_1$-$C_{10}$-Alkoxy, nieder-Alkylthio oder nieder--Alkoxy-nieder-alkoxy und $R^8$ und $R^9$ je Wasserstoff bedeuten.

**11.** Verbindungen gemäss einem der Ansprüche 1-10, worin R Isopropyl,Y Hydroxy, Acetyloxy oder Methoxyacetyloxy, $R^1$ , $R^4$ , $R^8$ und $R^9$ je Wasserstoff, $R^2$ und $R^3$ je nieder-Alkoxy oder $R^2$ Halogen und $R^3$ Wasserstoff, $R^5$ Wasserstoff oder $C_1$-$C_{10}$-Alkoxy, $R^6$ Wasserstoff, $C_1$-$C_{10}$-Alkoxy oder nieder-Alkoxy-nieder-alkoxy, $R^7$ Wasserstoff, Halogen, $C_1$-$C_{10}$-Alkoxy, nieder-Alkylthio oder nieder-Alkoxy-nieder-alkoxy und m und n je die Zahl 1 bedeuten.

**12.** 2-[2-[(3,4-Dimethoxyphenäthyl)methylamino]äthyl]-6-fluor-1,2,3,4-tetrahydro-1α-isopropyl-2α-naphthylmethoxyacetat.

**13.** 6,7-Dimethoxy-2-[2-[(3,4-dimethoxyphenäthyl)methylamino]äthyl]-1,2,3,4-tetrahydro-1α-isopropyl-2α-naphtyl-methoxyacetat.

**14.** 6,7-Dimethoxy-2-[2-[(3,4-dimethoxyphenätpyl)methylamino]äthyl]-1,2,3,4-tetrahydro-1α-isopropyl-2α-naphthylacetat.

**15.** [1S,2S]-2-[2-[(3,4-Dimethoxyphenäthyl)methylamino]-äthyl]-6-fluor-1,2,3,4-tetrahydro-1-isopropyl-2-naphthylmethoxyacetat.

**16.** Verbindungen der allgemeinen Formel

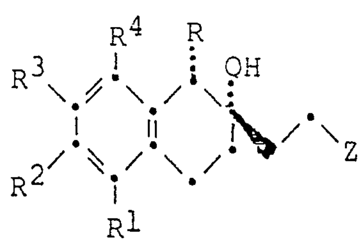

worin R Wasserstoff oder nieder-Alkyl, $R^1$, $R^2$, $R^3$ und $R^4$ unabhängig voneinander je Wasserstoff, Halogen oder nieder-Alkoxy oder zwei benachbarte Reste zusammen Methylendioxy oder Aethylendioxy und Z Hydroxy, Hydroxymethyl, Formyl, eine Abgangsgruppe oder die Gruppe $-CH_2-X$ bedeuten, worin X eine Abgangsgruppe bedeutet.

**17.** Tetrahydronaphthalinderivate gemäss einer der Ansprüche 1-15 zur Anwendung als therapeutische Wirkstoffe.

**18.** Tetrahydronaphthalinderivate gemäss einem der AnSprüche 1-15 zur Anwendung bei der Bekämpfung bzw. Verhütung von Angina pectoris, Ischämie, Arrhythmien und Bluthochdruck.

**19.** [1S,2S]-2-[2-[(3,4-Dimethoxyphenäthyl)methylamino]-äthyl]-6-fluor-1,2,3,4-tetrahydro-1-isopropyl-2-naphthylmethoxyacetat oder dessen Racemat zur Anwendung als therapeutischer Wirkstoff.

**20.** [1S,2S]-2-[2-[(3,4-Dimethoxyphenäthyl)methylamino]-äthyl]-6-fluor-1,2,3,4-tetrahydro-1-isopropyl-2-naphthylmethoxyacetat oder dessen Racemat zur Anwendung bei der Bekämpfung bzw. Verhütung von Angina pectoris, Ischämie, Arrhythmien und Bluthochdruck.

**21.** Verfahren zur Herstellung einer Verbindung gemäss einem der Ansprüche 1-15, dadurch gekennzeichnet, dass man

a) zur Herstellung von Verbindungen der Formel 1, worin Y Hydroxy bedeutet und die übrigen Symbole die in Anspruch 1 angegebene Bedeutung besitzen, eine Verbindung der allge meinen Formel

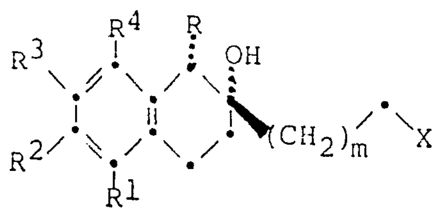

II

worin X eine Abgangsgruppe bedeutet und R, $R^1$, $R^2$, $R^3$, $R^4$ und m die in Anspruch 1 angegebene Bedeutung besitzen, mit einem Amin der allgemeinen Formel

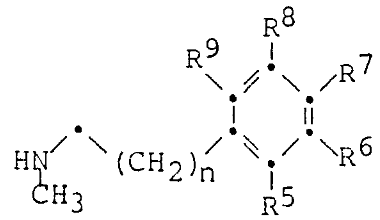

III

worin $R^5$, $R^6$, $R^7$, $R^8$, $R^9$ und n die in Anspruch 1 angegebene Bedeutung besitzen, umsetzt, oder

b) zur Herstellung von Verbindungen der Formel I, worin Y Hydroxy und m die Zahl 2 bedeuten und die übrigen Symbole die in Anspruch 1 angegebene Bedeutung besitzen, einen Aldehyd der allgemeinen Formel

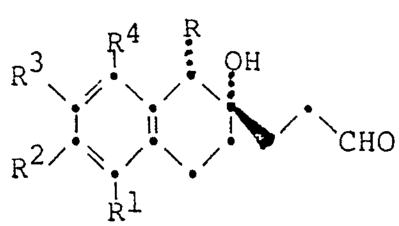

IV

worin R, $R^1$, $R^2$, $R^3$ und $R^4$ die in Anspruch 1 angegebene Bedeutung besitzen, mit einem Amin der obigen Formel III reduktiv aminiert, oder

c) zur Herstellung von Verbindungen der Formel I, worin Y nieder-Alkylcarbonyloxy, nieder-Alkoxy-nieder-alkylcarbonyloxy, nieder-Alkoxycarbonyloxy, nieder-Alkoxy-nieder-alkoxy carbonyloxy, nieder-Alkylthio-nieder-alkylcarbonyloxy oder gegebenenfalls substituiertes Benzylcarbonyloxy bedeutet und die übrigen Symbole die in Anspruch 1 angegebene Bedeutung besitzen, eine Verbindung der allgemeinen Formel

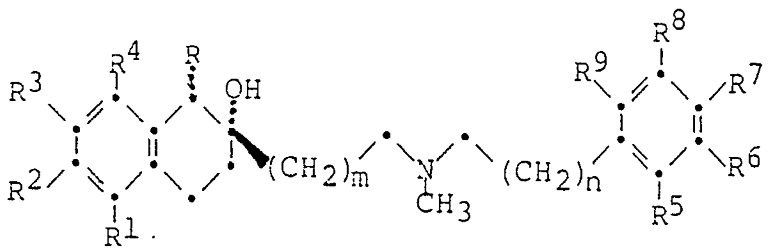

Ia

worin R, $R^1$, $R^2$, $R^3$, $R^4$, $R^5$, $R^6$, $R^7$, $R^8$, $R^9$, m und n die in Anspruch 1 angegebene Bedeutung besitzen, mit einem eine nieder-Alkylcarbonyl-, nieder-Alkoxy-nieder--alkylcarbonyl-, nieder-Alkoxycarbonyl-, nieder-Alkoxy-nieder-alkoxycarbonyl-, nieder-Alkylthio-nieder-alkylcarbonyl-oder gegebenenfalls substituierte Benzylcarbonyl-Gruppe abgebenden Acylierungsmittel umsetzt, und

d) erwünschtenfalls, ein erhaltenes Racemat in die optischen Antipoden auftrennt, und/oder

e) eine erhaltene Verbindung in ein pharmazeutisch verwendbares Säureadditionssalz überführt.

**22.** Arzneimittel, enthaltend ein Tetrahydronaphthalinderivat gemäss einem der Ansprüche 1-15 und ein therapeutisch inertes Excipiens.

**23.** Mittel zur Bekämpfung bzw. Verhütung von Angina pectoris, Ischämie, Arrhythmien und Bluthochdruck, enthaltend ein Tetrahydronaphthalinderivat gemäss einem der Ansprüche 1-15 und ein therapeutisch inertes Excipiens.

**24.** Arzneimittel, enthaltend [1S,2S]-2-[2-[(3,4--Dimethoxyphenäthyl)methylamino]äthyl]-6-fluor-1,2,3,4--tetrahydro-1-isopropyl-2-naphthyl-methoxyacetat oder dessen Racemat und ein therapeutisch inertes Excipiens.

**25.** Mittel zur Bekämpfung bzw. Verhütung von Angina pectoris, Ischämie, Arrhythmien und Bluthochdruck, enthaltend [1S,2S]-2-[2-[(3,4-Dimethoxyphenäthyl)methylamino]-äthyl]-6-fluor-1,2,3,4-tetrahydro-1-isopropyl -2-naphthyl--methoxyacetat oder dessen Racemat und ein therapeutisch inertes Excipiens.

**26.** Verwendung eines Tetrahydronaphthalinderivates gemäss einem der Ansprüche 1-15 zur Herstellung von Mitteln gegen Angina pectoris, Ischämie, Arrhythmien und/oder Bluthochdruck.

**27.** Verwendung von [1S,2S]-2-[2-[(3,4-Dimethoxyphenäthyl)methylamino]äthyl]-6-fluor-1,2,3,4-tetrahydro-1-isopropyl -2-naphthyl-methoxyacetat oder von dessen Racemat nach Anspruch 26.

Patentansprüche für folgenden Vertragsstaat:AT

**1.** Verfahren zur Herstellung von Tetrahydronaphthalin-derivaten der allgemeinen Formel

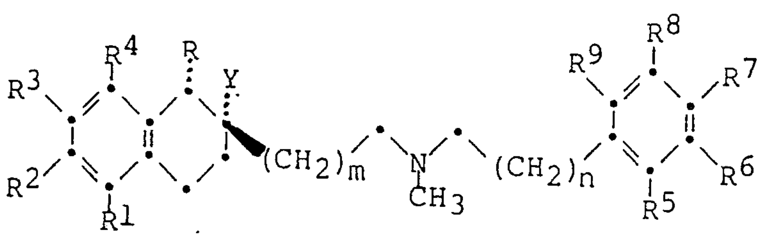

I

worin R Wasserstoff oder nieder-Alkyl, $R^1$, $R^2$, $R^3$ und $R^4$ unabhängig voneinander je Wasserstoff, Halogen oder nieder-Alkoxy oder zwei benachbarte Reste zusammen Methylendioxy oder Aethylendioxy, $R^5$, $R^6$, $R^7$, $R^8$ und $R^9$ unabhängig voneinander je Wasserstoff, Halogen, $C_1$-$C_{10}$-Alkoxy,

nieder Alkylthio, nieder--Alkoxy-nieder-alkoxy oder ω,ω,ω-Trifluor-nieder-alkoxy oder zwei benachbarte Reste zusammen Methylendioxy oder Aethylendioxy, Y Hydroxy, nieder-Alkylcarbonyloxy, nieder-Alkoxy-nieder-alkylcarbonyloxy, nieder-Alkoxycarbonyloxy, nieder-Alkoxy-nieder-alkoxycarbonyloxy, nieder-Alkylthio-nieder-alkylcarbonyloxy oder gegebenenfalls substituiertes Benzylcarbonyloxy, m die Zahl 1 oder 2 und n die Zahl 1, 2 oder 3 bedeuten, in Form von Racematen und optischen Antipoden, sowie pharmazeutisch verwendbaren Säureadditionssalzen davon, dadurch gekennzeichnet, dass man

a) zur Herstellung von Verbindungen der Formel I, worin Y Hydroxy bedeutet und die übrigen Symbole die oben angegebene Bedeutung besitzen, eine Verbindung der allgemeinen Formel

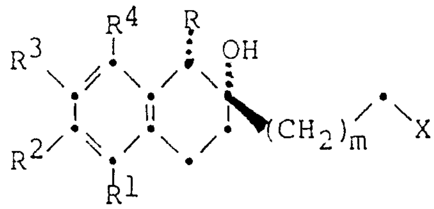

II

worin X eine Abgangsgruppe bedeutet und R, $R^1$, $R^2$, $R^3$, $R^4$ und m die oben angegebene Bedeutung besitzen, mit einem Amin der allgemeinen Formel

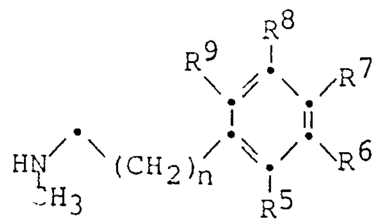

III

worin $R^5$, $R^6$ , $R^7$ , $R^8$, $R^9$ und n die oben angegebene Bedeutung besitzen, umsetzt, oder

b) zur Herstellung von Verbindungen der Formel I, worin Y Hydroxy und m die Zahl 2 bedeuten und die übrigen Symbole die oben angegebene Bedeutung besitzen, einen Aldehyd der allgemeinen Formel

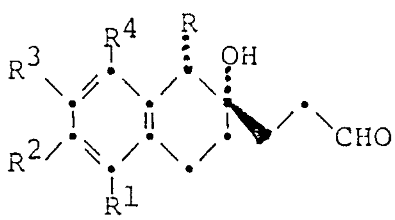

IV

worin R, $R^1$ , $R^2$ , $R^3$ und $R^4$ die oben angegebene Bedeutung besitzen, mit einem Amin der obigen Formel III reduktiv aminiert, oder

c) zur Herstellung von Verbindungen der Formel I, worin Y nieder-Alkylcarbonyloxy, nieder-Alkoxy-nieder-alkylcarbonyloxy, nieder-Alkoxycarbonyloxy, nieder-Alkoxy-nieder-alkoxycarbonyloxy, nieder-Alkylthio-nieder-alkylcarbonyloxy oder gegebenenfalls substituiertes Benzylcarbonyloxy bedeutet und die übrigen Symbole die oben angegebene Bedeutung besitzen, eine Verbindung der allgemeinen Formel

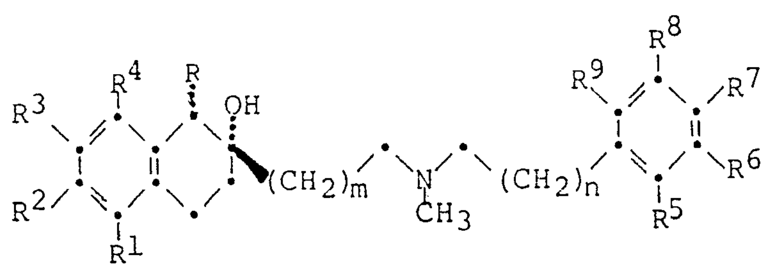

Ia

worin R, $R^1$, $R^2$, $R^3$, $R^4$, $R^5$, $R^6$, $R^7$, $R^8$, $R^9$, m und n die oben angegebene Bedeutung besitzen, mit einem eine nieder-Alkylcarbonyl-, nieder-Alkoxy-nieder--alkylcarbonyl-, nieder-Alkoxycarbonyl-, nieder-Alkoxy-nieder-alkoxycarbonyl-, nieder-Alkylthio-nieder-alkylcarbonyl-oder gegebenenfalls substituierte Benzylcarbonyl-Gruppe abgebenden Acylierungsmittel umsetzt, und

d) erwünschtenfalls, ein erhaltenes Racemat in die optischen Antipoden auftrennt, und/oder

e) eine erhaltene Verbindung in ein pharmazeutisch verwendbares Säureadditionssalz überführt.

**2.** Verfahren gemäss Anspruch 1, worin R nieder-Alkyl bedeutet.

**3.** Verfahren gemäss Anspruch 2, worin R Isopropyl bedeutet.

**4.** Verfahren gemäss einem der Ansprüche 1-3, worin Y Hydroxy, nieder-Alkylcarbonyloxy oder nieder-Alkoxy-nieder-alkylcarbonyloxy bedeutet.

**5.** Verfahren gemäss Anspruch 4, worin Y Hydroxy, Acetyloxy oder Methoxyacetyloxy bedeutet.

**6.** Verfahren gemäss einem der Ansprüche 1-5, worin m und n je die Zahl 1 bedeuten.

**7.** Verfahren gemäss einem der Ansprüche 1-6, worin zwei der Substituenten $R^1$, $R^2$, $R^3$ und $R^4$ Wasserstoff und die beiden anderen unabhängig voneinander je Wasserstoff, Halogen oder nieder-Alkoxy bedeuten.

**8.** Verfahren gemäss Anspruch 7, worin $R^1$ und $R^4$ je Wasserstoff, $R^2$ und $R^3$ je nieder-Alkoxy oder $R^2$ Halogen und $R^3$ Wasserstoff bedeuten.

**9.** Verfahren gemäss einem der Ansprüche 1-8, worin zwei der Substituenten $R^5$, $R^6$, $R^7$, $R^8$ und $R^9$ Wasserstoff und die drei anderen unabhängig voneinander je Wasserstoff, Halogen, $C_1$-$C_{10}$-Alkoxy, nieder-Alkylthio oder nieder-Alkoxy-nieder-alkoxy bedeuten.

**10.** Verfahren gemäss Anspruch 9, worin $R^5$ Wasserstoff oder $C_1$-$C_{10}$-Alkoxy, $R^6$ Wasserstoff, $C_1$-$C_{10}$--Alkoxy oder nieder-Alkoxy-nieder-alkoxy, $R^7$ Wasserstoff, Halogen, $C_1$-$C_{10}$-Alkoxy, nieder-Alkylthio oder nieder--Alkoxy-nieder-alkoxy und $R^8$ und $R^9$ je Wasserstoff bedeuten.

**11.** Verfahren gemäss einem der Ansprüche 1-10, worin R Isopropyl,Y Hydroxy, Acetyloxy oder Methoxyacetyloxy, $R^1$, $R^4$, $R^8$ und $R^9$ je Wasserstoff, $R^2$ und $R^3$ je nieder-Alkoxy oder $R^2$ Halogen und $R^3$ Wasserstoff, $R^5$ Wasserstoff oder $C_1$-$C_{10}$-Alkoxy, $R^6$ Wasserstoff, $C_1$-$C_{10}$-Alkoxy oder nieder-Alkoxy-nieder-alkoxy, $R^7$ Wasserstoff, Halogen, $C_1$-$C_{10}$-Alkoxy, nieder-Alkylthio oder nieder-Alkoxy-nieder-alkoxy und m und n je die Zahl 1 bedeuten.

**12.** Verfahren gemäss Anspruch 1, dadurch gekennzeichnet, dass man 2-[2-[(3,4-Dimethoxyphenäthyl)-methylamino]-äthyl]-6-fluor-1,2,3,4-tetrahydro-1α-isopropyl -2α-naphthylmethoxyacetat herstellt.

**13.** Verfahren gemäss Anspruch 1, dadurch gekennzeichnet, dass man 6,7-Dimethoxy-2-[2-[(3,4-dimethoxyphenäthyl)-methylamino]äthyl]-1,2,3,4-tetrahydro-1α-isopropyl-2α-naphtylmethoxyacetat herstellt.

**14.** Verfahren gemäss Anspruch 1, dadurch gekennzeichnet, dass man 6,7-Dimethoxy-2-[2-[(3,4-dimethoxyphenäthyl)-methylamino]äthyl]-1,2,3,4-tetrahydro-1α-isopropyl-2α--naphthylacetat herstellt.

**15.** Verfahren gemäss Anspruch 1, dadurch gekennzeichnet, dass man [1S,2S]-2-[2-[(3,4-Dimethoxyphenäthyl)methylamino]äthyl]-6-fluor-1,2,3,4-tetrahydro-1-isopropyl-2-naphthylmethoxyacetat herstellt.

**16.** Verwendung eines Tetrahydronaphthalinderivates gemäss einem der Ansprüche 1-15 zur Herstellung von Mitteln gegen Angina pectoris, Ischämie, Arrhythmien und/oder Bluthochdruck.

**17.** Verwendung von [1S,2S]-2-[2-[(3,4-Dimethoxyphenäthyl)methylamino]äthyl]-6-fluor-1,2,3,4-tetrahydro-1-isopropyl -2-naphthyl-methoxyacetat oder von dessen Racemat nach Anspruch 16.

## Claims

1. Tetrahydronaphthalene derivatives of the general formula

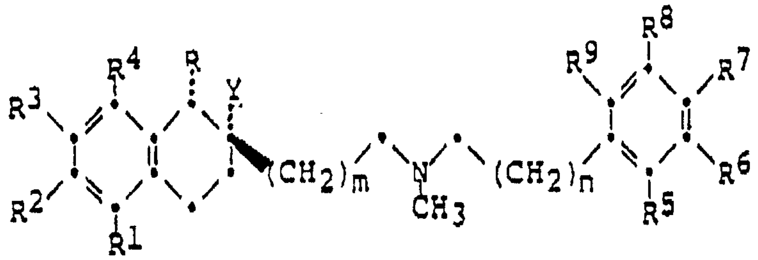

I

wherein R signifies hydrogen or lower-alkyl, $R^1$, $R^2$, $R^3$ and $R^4$ each independently signify hydrogen, halogen or lower-alkoxy or two adjacent residues together signify methylenedioxy or ethylenedioxy, $R^5$, $R^6$, $R^7$, $R^8$ and $R^9$ each independently signify hydrogen, halogen, $C_1$-$C_{10}$-alkoxy, lower alkylthio, lower-alkoxy-lower-alkoxy or ω,ω,ω-trifluoro-lower-alkoxy or two adjacent residues together signify methylenedioxy or ethylenedioxy, Y signifies hydroxy, lower-alkylcarbonyloxy, lower-alkoxy-lower-alkylcarbonyloxy, lower-alkoxycarbonyloxy, lower-alkoxy-lower-alkoxycarbonyloxy, lower--alkylthio-lower-alkylcarbonyloxy or optionally substituted benzylcarbonyloxy, m signifies the number 1 or 2 and n signifies the number 1, 2 or 3, in the form of racemates and optical antipodes, as well as pharmaceutically usable acid addition salts thereof,

2. Compounds in accordance with claim 1. wherein R signifies lower-alkyl.

3. Compounds in accordance with claim 2. wherein R signifies isopropyl.

4. Compounds in accordance with any one of claims 1-3. wherein y signifies hydroxy, lower-alkylcarbonyloxy of lower-alkoxy-lower-alkylcarbonyloxy,

5. Compounds in accordance with claim 4, wherein Y signifies hydroxy, acetoxy or methoxyacetoxy,

6. Compounds in accordance with any one of claims 1-5, wherein m and n each signify the number 1.

7. Compounds in accordance with any one of claims 1-6, wherein two of the substituents $R^1$ , $R^2$, $R^3$ and $R^4$ signify hydrogen and the other two each independently signify hydrogen, halogen or lower-alkoxy.

8. Compounds in accordance with claim 7, wherein $R^1$ and $R^4$ each signify hydrogen and $R^2$ and $R^3$ each signify lower-alkoxy or $R^2$ signifies halogen and $R^3$ signifies hydrogen.

9. Compounds in accordance with any one of claims 1-8. wherein two of the substituents $R^5$, $R^6$, $R^7$, $R^8$ and $R^9$ signify hydrogen and the other three each independently signify hydrogen, halogen, $C_1$-$C_{10}$-alkoxy, lower-alkylthio or lower-alkoxy-lower-alkoxy,

10. Compounds in accordance with claim 9. wherein $R^5$ signifies hydrogen or $C_1$-$C_{10}$-alkoxy, $R^6$ signifies hydrogen, $C_1$-$C_{10}$-alkoxy or lower-alkoxy-lower-alkoxy, $R^7$ signifies hydrogen, halogen. $C_1$-$C_{10}$-alkoxy, lower-alkylthio or lower-alkoxy-lower-alkoxy and $R^8$ and $R^9$ each signify hydrogen.

11. Compounds in accordance with any one of claims 1-10, wherein R signifies isopropyl, Y signifies hydroxy, acetoxy or methoxyacetoxy, $R^1$, $R^4$, $R^8$ and $R^9$ each signify hydrogen, $R^2$ and $R^3$ each signify lower-alkoxy or $R^2$ signifies halogen and $R^3$ signifies hydrogen, $R^5$ signifies hydrogen or $C_1$-$C_{10}$-alkoxy, $R^6$ signifies hydrogen, $C_1$-$C_{10}$-alkoxy or lower-alkoxy-lower-alkoxy, $R^7$ signifies hydrogen, halogen, $C_1$-$C_{10}$-alkoxy, lower-alkylthio or lower-alkoxy-lower-alkoxy and m and n each signify the number 1.

12. 2-[2-[(3,4-Dimethoxyphenethyl)methylamino]ethyl]-6-fluoro-1,2,3,4-tetrahydro-1α-isopropyl-2α-naphthyl methoxyacetate.

13. 6,7-Dimethoxy-2-[2-[(3,4-dimethoxyphenethyl)methylamino]ethyl]-1,2,3,4-tetrahydro-1α-isopropyl-2α-naphthyl methoxyacetate.

14. 6,7-Dimethoxy-2-[2-[(3,4-dimethoxyphenethyl)methylamino]ethyl]-1,2,3,4-tetrahydro-1α-isopropyl-2α-naphthyl acetate.

15. [1S,2S]-2-[2-[(3,4-Dimethoxyphenethyl)methylamino]-ethyl]-6-fluoro-1,2,3,4-tetrahydro-1-isopropyl-2-naphthyl methoxyacetate.

16. Compounds of the general formula

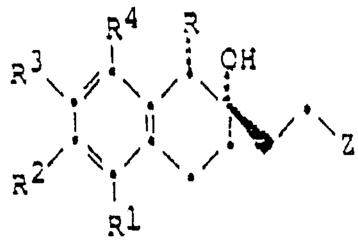

wherein R signifies hydrogen or lower-alkyl, $R^1$, $R^2$, $R^3$ and $R^4$ each independently signify hydrogen, halogen or lower-alkoxy or two adjacent residues together signify methylenedioxy or ethylenedioxy and Z signifies hydroxy, hydroxymethyl, formyl, a leaving group or the group $-CH_2-X$ in which X signifies a leaving group.

17. Tetrahydronaphthalene derivatives in accordance with any one of claims 1-15 for use as therapeutically active substances.

18. Tetrahydronaphthalene derivatives in accordance with any one of claims 1-15 for use in the control or prevention of angina pectoris, ischaemia, arrhythmias and high blood pressure.

19. [1S,2S]-2-[2-[(3,4-Dimethoxyphenethyl)methylamino]-ethyl]-6-fluoro-1,2,3,4-tetrahydro-1-isopropyl-2-naphthyl methoxyacetate or its racemate for use as a therapeutically active substance.

20. [1s,2s]-2-[2-[(3,4-Dimethoxyphenethyl)methylamino]-ethyl]-6-fluoro-1,2,3,4,-tetrahydro-1-isopropyl-2-naphthyl methoxyacetate or its racemate for use in the control or prevention of angina pectoris, ischaemia, arrhythmias and high blood pressure.

21. A process for the manufacture of a compound in accordance with any one of claims 1-15, characterized by

a) for the manufacture of compounds of formula I in which Y signifies hydroxy and the remaining symbols have the significance given in claim 1, reacting a compound of the general formula

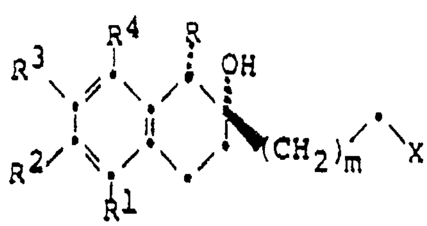

II

wherein X signifies a leaving group and R, $R^1$, $R^2$, $R^3$, $R^4$ and m have the significance given in claim 1, with an amine of the general formula

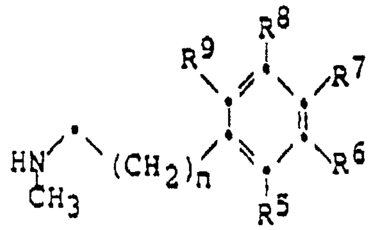

III

wherein $R^5$, $R^6$, $R^7$, $R^8$, $R^9$ and n have the significance given in claim 1, or

b) for the manufacture of compounds of formula I in which Y signifies hydroxy and m signifies the number 2 and the remaining symbols have the significance given in claim 1, reductively aminating an aldehyde of the general formula

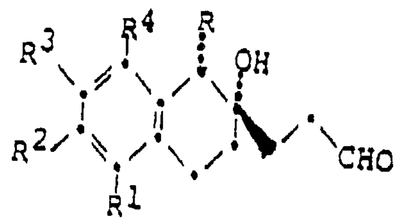

IV

wherein R, $R^1$, $R^2$, $R^3$ and $R^4$ have the significance given in claim 1, with an amine of formula III above, or

c) for the manufacture of compounds of formula I In which Y signifies lower-alkylcarbonyloxy, lower-alkoxy-lower-alkylcarbonyloxy, lower-alkoxycarbonyloxy, lower-alkoxy-lower-alkoxycarbonyloxy, lower-alkylthio-lower-alkylcarbonyloxy or optionally substituted benzylcarbonyloxy and the remaining symbols have the significance given in claim 1, reacting a compound of the general formula

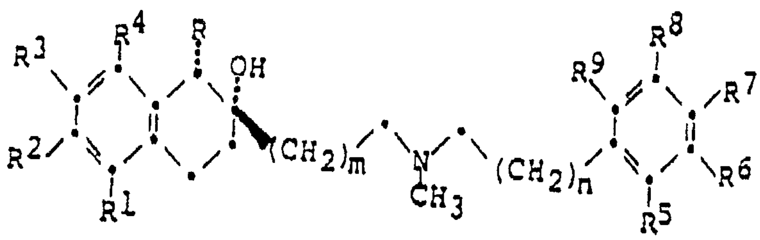

Ia

wherein R, $R^1$, $R^2$, $R^3$, $R^4$, $R^5$, $R^6$, $R^7$, $R^8$, $R^9$, m and n have the significance given in claim 1, with an acylating agent yielding a lower-alkylcarbonyl, lower-alkoxy-lower-alkylcarbonyl, lower-alkoxycarbonyl, lower-alkoxy-lower-alkoxycarbonyl, lower-alkylthio-lower--alkylcarbonyl or optionally substituted benzylcarbonyl group, and

d) if desired, resolving a racemate obtained into the optical antipodes, and/or

e) converting a compound obtained into a pharmaceutically usable acid addition salt.

**22.** A medicament containing a tetrahydronaphthalene derivative in accordance with any one of claims 1-15 and a therapeutically inert excipient.

**23.** A medicament for the control or prevention of angina pectoris, ischaemia, arrhythmias and high blood pressure, containing a tetrahydronaphthalene derivative in accordance with any one of claims 1-15 and a therapeutically inert excipient.

**24.** A medicament containing [1S,2S]-2-[2-[(3,4-dimethoxyphenethyl)methylamino]ethyl]-6-flouro-1,2,3,4-tetrahydro-1-isopropyl-2-naphthyl methoxyacetate or its racemate and a therapeutically inert excipient.

**25.** A medicament for the control or prevention of angina pectoris, ischaemia, arrhythmias and high blood pressure, containing [1S,2S]-2-[2-[(3,4 -dimethoxyphenethyl)methylamino]ethyl]-6-fluoro-1,2,3,4-tetrahydro-1-isopropyl-2-naphthyl methoxyacetate or its racemate and a therapeutically inert excipient.

**26.** The use of a tetrahydronaphthalene derivative in accordance with any one of claims 1-15 for the manufacture of medicaments against angina pectoris, ischaemia, arrhythmias and/or high blood pressure.

**27.** The use of [1S,2S]-2-[2-[(3,4-dimethoxyphenethyl)methylamino]ethyl]-6-fluoro-1,2,3,4-tetrahydro-1-isopropyl-2-naphthyl methoxyacetate or of its racemate according to claim 26.

Claims for the following Contracting State: AT

**1.** A process for the manufacture of tetrahydronaphthalene derivatives of the general formula

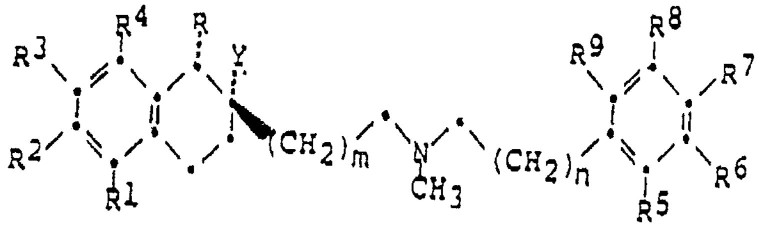

I

wherein R signifies hydrogen or lower-alkyl, $R^1$, $R^2$, $R^3$ and $R^4$ each independently signify hydrogen, halogen or lower-alkoxy or two adjacent residues together signify methylenedioxy or ethylenedioxy, $R^5$, $R^6$, $R^7$, $R^8$ and $R^9$ each independently signify hydrogen, halogen, $C_1$-$C_{10}$-alkoxy, lower alkylthio, lower-alkoxy-lower-alkoxy or ω,ω,ω-trifluoro-lower-alkoxy or two adjacent residues together signify methylenedioxy or ethylenedioxy, y signifies hydroxy, lower-alkylcarbonyloxy, lower-alkoxy-lower-alkylcarbonyloxy, lower-alkoxycarbonyloxy, lower-alkoxy-lower-alkoxycarbonyloxy, lower-alkylthio-lower-alkylcarbonyloxy or optionally substituted benzylcarbonyloxy, m signifies the number 1 or 2 and n signifies the number 1, 2 or 3, in the form of racemates and optical antipodes, as well as pharmaceutically usable acid addition salts thereof, characterized by

a) for the manufacture of compounds of formula I in which Y signifies hydroxy and the remaining symbols have the significance given above, reacting a compound of the general formula

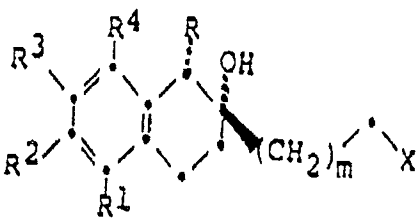

II

wherein X signifies a leaving group and R, $R^1$, $R^2$, $R^3$, $R^4$ and m have the significance given above, with an amine of the general formula

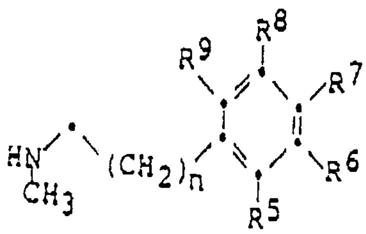

III

wherein $R^5$, $R^6$, $R^7$, $R^8$, $R^9$ and n have the significance given above, or

b) for the manufacture of compounds of formula I in which Y signifies hydroxy and m signifies the number 2 and the remaining symbols have The significance given above, reductively aminating an aldehyde of the general formula

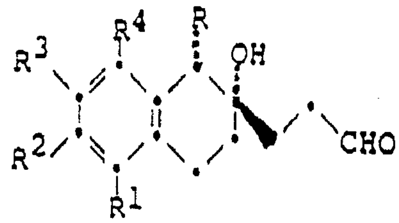

IV

wherein R, $R^1$, $R^2$, $R^3$ and $R^4$ have the significance given above, with an amine of formula III above, or

c) for the manufacture of compounds of formula I in which Y signifies lower-alkylcarbonyloxy, lower-alkoxy-lower-alkylcarbonyloxy, lower-alkoxycarbonyloxy, lower-alkoxy -lower-alkoxycarbonyloxy, lower-alkylthio-lower-alkylcarbonyloxy or optionally substituted benzylcarbonyloxy and the remaining symbols have the significance given above, reacting a compound of the general formula

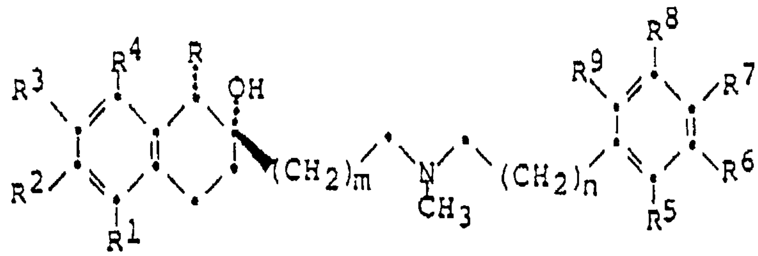

Ia

wherein R, $R^1$, $R^2$, $R^3$, $R^4$, $R^5$, $R^6$, $R^7$, $R^8$, $R^9$, m and n have the significance given above, with an acylating agent yielding a lower-alkylcarbonyl, lower-alkoxy-lower-alkylcarbonyl, lower-alkoxycarbonyl, lower-alkoxy-lower-alkoxycarbonyl, lower-alkylthio-lower-alkylcarbonyl or optionally substituted benzylcarbonyl group, and

d) if desired, resolving a racemate obtained into the optical antipodes, and/or

e) converting a compound obtained into a pharmaceutically usable acid addition salt.

**2.** A process in accordance with claim 1, wherein R signifies lower-alkyl.

**3.** A process in accordance with claim 2, wherein R signifies isopropyl.

**4.** A process in accordance with any one of claims 1-3. wherein Y signifies hydroxy, lower-alkylcarbonyloxy or lower-alkoxy-lower-alkylcarbonyloxy.

**5.** A process in accordance with claim 4, wherein Y signifies hydroxy, acetoxy or methoxyacetoxy.

**6.** A process in accordance with any one of claims 1-5, wherein m and n each signify the number 1.

**7.** A process in accordance with any one of claims 1-6, wherein two of the substituents $R^1$, $R^2$, $R^3$ and $R^4$ signify hydrogen and the other two each independently signify hydrogen, halogen or lower-alkoxy.

**8.** A process in accordance with claim 7, wherein $R^1$ and $R^4$ each signify hydrogen and $R^2$ and $R^3$ each signify lower-alkoxy or $R^2$ signifies halogen and $R^3$ signifies hydrogen.

**9.** A process in accordance with any one of claims 1-8. wherein two of the substituents $R^5$ , $R^6$, $R^7$, $R^8$ and $R^9$ signify hydrogen and the other three each independently signify hydrogen, halogen, $C_1$-$C_{10}$--alkoxy, lower-alkylthio or lower-alkoxy-lower-alkoxy.

**10.** A process in accordance with claim 9, wherein $R^5$ signifies hydrogen or $C_1$-$C_{10}$-alkoxy, $R^6$ signifies hydrogen, $C_1$-$C_{10}$-alkoxy or lower-alkoxy-lower-alkoxy, $R^7$ signifies hydrogen, halogen, $c_1$-$C_{10}$-alkoxy, lower-alkylthio or lower-alkoxy-lower-alkoxy and $R^8$ and $R^9$ each signify hydrogen.

**11.** A process in accordance with any one of claims 1-10, wherein R signifies isopropyl, Y signifies hydroxy, acetoxy or methoxyacetoxy, $R^1$ , $R^4$, $R^8$ and $R^9$ each signify hydrogen, $R^2$ and $R^3$ each

signify lower-alkoxy or $R^2$ signifies halogen and $R^3$ signifies hydrogen, $R^5$ signifies hydrogen or $C_1$-$C_{10}$-alkoxy, $R^6$ signifies hydrogen, $C_1$-$C_{10}$-alkoxy or lower-alkoxy-lower-alkoxy, $R^7$ signifies hydrogen, halogen, $C_1$-$C_{10}$-alkoxy, lower-alkylthio or lower-alkoxy-lower-alkoxy and m and n each signify the number 1.

**12.** A process in accordance with claim 1. characterized in that 2-[2-[(3,4-dimethoxyphenethyl)-methylamino]-ethyl]-6-fluoro-1,2,3,4-tetrahydro-1α-isopropyl-2α--naphthyl methoxyacetate is manufactured.

**13.** A process in accordance with claim 1, characterized in that 6,7-dimethoxy-2-[2-[(3,4-dimethoxyphenethyl)-methylamino]ethyl]-1,2,3,4-tetrahydro-1α-isopropyl-2α--naphthylmethoxyacetate is manufactured.

**14.** A process in accordance with claim 1, characterized in that 6,7-dimethoxy-2-[2-[(3,4-dimethoxyphenethyl)-methylamino]ethyl]-1,2,3,4-tetrahydro-1α-isopropyl-2α-naphthylacetate is manufactured.

**15.** A process in accordance with claim 1, characterized in that [1S,2S]-2-[2-[(3,4-dimethoxyphenethyl)-methylamino]-ethyl]-6-fluoro-1,2,3,4-tetrahydro-1-isopropyl-2-naphthyl methoxyacetate is manufactured.

**16.** The use of a tetrahydronaphthalene derivative in accordance with any one of claims 1-15 for the manufacture of medicaments against angina pectoris, ischaemia. arrhythmias and/or high blood pressure.

**17.** The use of [1S,2S]-2-[2-[(3,4-dimethoxyphenethyl)methylamino]ethyl]-6-fluoro-1,2,3,4-tetrahydro-1--isopropyl-2-naphthyl methoxyacetate or of its racemate according to claim 16.

## Revendications

**1.** Dérivés du tétrahydronaphtalène répondant à la formule générale

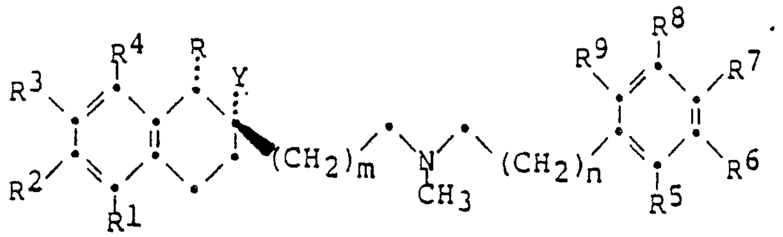

I

dans laquelle R représente l'hydrogène ou un groupe alkyle inférieur, $R^1$, $R^2$, $R^3$ et $R^4$ représentent chacun, indépendamment les uns des autres, l'hydrogène, un halogène ou un groupe alcoxy inférieur ou bien deux groupes voisins forment ensemble un groupe méthylène-dioxy ou éthylène-dioxy, $R^5$, $R^6$, $R^7$, $R^8$ et $R^9$ représentent chacun, indépendamment les uns des autres, l'hydrogène, un halogène, un groupe alcoxy en C 1-C 10, alkylthio inférieur, (alcoxy inférieur)-alcoxy inférieur ou oméga,oméga,oméga-trifluoro-alcoxy inférieur ou bien deux groupes voisins forment ensemble un groupe méthylène-dioxy ou éthylene-dioxy, Y représente un groupe hydroxy, (alkyle inférieur)-carbonyloxy, (alcoxy inférieur)-(alkyle inférieur)-carbonyloxy, (alcoxy inférieur)-carbonyloxy, (alcoxy inférieur)-(alcoxy inférieur)-carbonyloxy, (alkylthio inférieur)-(alkyle inférieur)-carbonyloxy ou un groupe benzylcarbonyloxy éventuellement substitué, m est égal à 1 ou 2 et n à 1, 2 ou 3,
à l'état de racémates et d'antipodes optiques, et leurs sels formés par addition avec des acides acceptables pour l'usage pharmaceutique.

**2.** Composés selon la revendication 1, dans lesquels R représente un groupe alkyle inférieur.

**3.** Composés selon la revendication 2, dans lesquels R représente un groupe isopropyle.

**4.** Composés selon l'une des revendications 1 à 3, dans lesquels Y représente un groupe hydroxy, (alkyle

inférieur)-carbonyloxy ou (alcoxy inférieur)-(alkyle inférieur)-carbonyloxy.

5. Composés selon la revendication 4, dans lesquels Y représente un groupe hydroxy, acétyloxy ou méthoxyacétyloxy.

6. Composés selon l'une des revendications 1 à 5, dans lesquels m et n sont tous deux égaux à 1.

7. Composés selon l'une des revendications 1 à 6, dans lesquels deux des symboles $R^1$, $R^2$, $R^3$ et $R^4$ représentent l'hydrogène et les deux autres, indépendamment l'un de l'autre, l'hydrogène, des halogènes ou des groupes alcoxy inférieurs.

8. Composés selon la revendication 7, dans lesquels $R^1$ et $R^4$ représentent tous deux l'hydrogène, $R^2$ et $R^3$ représentent chacun un groupe alcoxy inférieur ou bien $R^2$ représente un halogène et $R^3$ l'hydrogène.

9. Composés selon l'une des revendications 1 à 8, dans lesquels deux des symboles $R^5$, $R^6$, $R^7$, $R^8$ et $R^9$ représentent l'hydrogène et les trois autres représentent chacun, indépendamment les uns des autres, l'hydrogène, un halogène, un groupe alcoxy en C 1-C 10, alkylthio inférieur ou (alcoxy inférieur)-alcoxy inférieur.

10. Composés selon la revendication 9, dans lesquels $R^5$ représente l'hydrogène ou un groupe alcoxy en C 1-C 10, $R^6$ représente l'hydrogène, un groupe alcoxy en C 1-C 10 ou (alcoxy inférieur)-alcoxy inférieur, $R^7$ représente l'hydrogène, un halogène, un groupe alcoxy en C 1-C 10, alkylthio inférieur ou (alcoxy inférieur)-alcoxy inférieur et $R^8$ et $R^9$ représentent chacun l'hydrogène.

11. Composés selon l'une des revendications 1 à 10, dans lesquels R représente un groupe isopropyle, Y représente un groupe hydroxy, acétyloxy ou méthoxyacétyloxy, $R^1$ $R^4$ $R^8$ et $R^9$ représentent chacun l'hydrogène, $R^2$ et $R^3$ représentent chacun un groupe alcoxy inférieur ou bien $R^2$ représente un halogène et $R^3$ l'hydrogène, $R^5$ représente l'hydrogène ou un groupe alcoxy en C 1-C 10, $R^6$ représente l'hydrogène, un groupe alcoxy en C 1-C 10 ou (alcoxy inférieur)-alcoxy inférieur, $R^7$ représente l'hydrogène, un halogène, un groupe alcoxy en C 1-C 10, alkylthio inférieur ou (alcoxy inférieur)-alcoxy inférieur et m et n sont chacun égaux à 1.

12. Le méthoxyacétate de 2-[2-[(3,4-diméthoxyphénéthyl)-méthylamino]-éthyl]-6-fluoro-1,2,3,4-tétrahydro-1 alpha-isopropyl-2 alpha-naphtyle.

13. Le méthoxyacétate de 6,7-diméthoxy-2-[2-[(3,4-diméthoxyphénéthyl)-méthylamino]-éthyl]-l,2,3,4-tétrahydro-1 alpha-isopropyl-2 alpha-naphtyle.

14. L'acétate de 6,7-diméthoxy-2-[2-[(3,4-diméthoxyphénéthyl)-méthylamino]-éthyl]-l,2,3,4-tétrahydro-1 alpha-isopropyl-2 alpha-naphtyle.

15. Le méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle.

16. Composés de formule générale

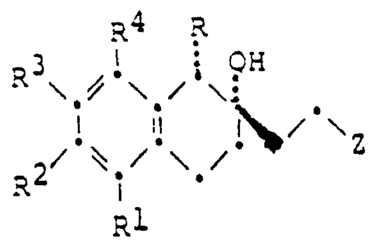

dans laquelle R représente l'hydrogène ou un groupe alkyle inférieur, $R^1$, $R^2$, $R^3$ et $R^4$ représentent chacun, indépendamment les uns des autres, l'hydrogène, un halogène ou un groupe alcoxy inférieur

ou bien deux groupes voisins forment ensemble un groupe méthylène-dioxy ou éthylène-dioxy et Z représente un groupe hydroxy, hydroxyméthyle, formyle, un groupe éliminable ou le groupe $-CH_2-X$ dans lequel X représente un groupe éliminable.

**17.** Dérivés du tétrahydronaphtalène selon l'une des revendications 1 à 15, pour l'utilisation en tant que substances actives thérapeutiques.

**18.** Dérivés du tétrahydronaphtalène selon l'une des revendications 1 à 15, pour l'utilisation dans le traitement ou la prévention de l'angine de poitrine, de l'ischémie, des arythmies et de l'hypertension sanguine.

**19.** Le méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle ou son racémate pour l'utilisation en tant que substance active thérapeutique.

**20.** Le méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle ou son racémate pour l'utilisation dans le traitement ou la prévention de l'angine de poitrine, de l'ischémie, des arythmies et de l'hypertension sanguine.

**21.** Procédé de préparation d'un composé selon l'une des revendications 1 à 15, caractérisé en ce que :
a) pour la préparation des composés de formule I dans laquelle Y représente un groupe hydroxy et les autres symboles ont les significations indiquées dans la revendication 1, on fait réagir un composé de formule générale

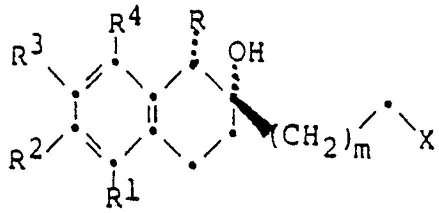

II

dans laquelle X représente un groupe éliminable et R, $R^1$, $R^2$,$R^3$,$R^4$ et m ont les significations indiquées dans la revendication 1,
avec une amine de formule générale

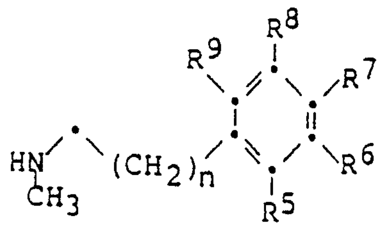

III

dans laquelle $R^5$, $R^6$, $R^7$, $R^8$, $R^9$ et n ont les significations indiquées dans la revendication 1, ou bien
b) pour la préparation des composés de formule I dans laquelle Y représente un groupe hydroxy et m est égal à 2, les autres symboles ayant les significations indiquées dans la revendication 1, on soumet un aldéhyde de formule générale

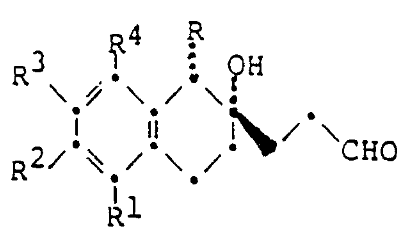

IV

dans laquelle R, $R^1$, $R^2$, $R^3$ et $R^4$ ont les significations indiquées dans la revendication 1, à amination réductive par une amine de formule III ci-dessus, ou bien

c) pour la préparation des composés de formule I dans laquelle Y représente un groupe (alkyle inférieur)-carbonyloxy, (alcoxy inférieur)-(alkyle inférieur)-carbonyloxy, (alcoxy inférieur)-carbonyloxy, (alcoxy inférieur)-(alcoxy inférieur)-carbonyloxy, (alkylthio inférieur)-(alkyle inférieur)-carbonyloxy ou un groupe benzylcarbonyloxy éventuellement substitué et les autres symboles ont les significations indiquées dans la revendication 1, on fait réagir un composé de formule générale

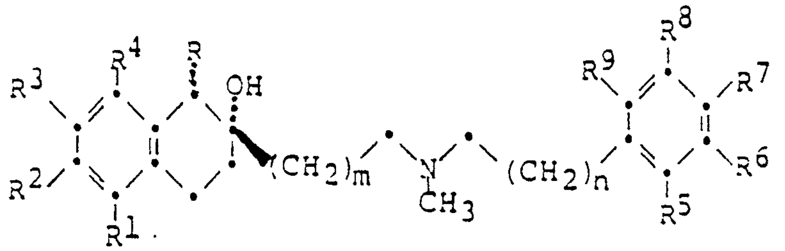

Ia

dans laquelle R, $R^1$, $R^2$, $R^3$, $R^4$, $R^5$, $R^6$, $R^7$, $R^8$, $R^9$ et m et n ont les significations indiquées dans la revendication 1,

avec un agent acylant apportant un groupe (alkyle inférieur)-carbonyle, (alcoxy inférieur)-(alkyle inférieur)-carbonyle, (alcoxy inférieur)-carbonyle, (alcoxy inférieur)-(alcoxy inférieur)-carbonyle, (alkylthio inférieur)(alkyle inférieur)-carbonyle ou un groupe benzylcarbonyle éventuellement substitué, et

d) si on le désire, on résout un racémate ainsi obtenu en les antipodes optiques, et/ou

e) on convertit un composé ainsi obtenu en un sel par addition avec un acide acceptable pour l'usage pharmaceutique.

**22.** Médicament contenant un dérivé du tétrahydronaphtalène selon l'une des revendications 1 à 15 et un excipient inerte du point de vue thérapeutique.

**23.** Médicament pour le traitement ou la prévention de l'angine de poitrine, de l'ischémie, des arythmies et de l'hypertension sanguine, contenant un dérivé du tétrahydronaphtalène selon l'une des revendications 1 à 15 et un excipient inerte du point de vue thérapeutique.

**24.** Médicament contenant le méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle ou son racémate et un excipient inerte du point de vue thérapeutique.

**25.** Médicament pour le traitement ou la prévention de l'angine de poitrine, de l'ischémie, des arythmies et de l'hypertension sanguine, contenant le méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle ou son racémate et un excipient inerte du point de vue thérapeutique.

**26.** Utilisation d'un dérivé du tétrahydronaphtalène selon l'une des revendications 1 à 15 pour la préparation de médicaments contre l'angine de poitrine, l'ischémie, les arythmies et/ou l'hypertension sanguine.

**27.** Utilisation du méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle ou de son racémate selon la revendication 26.

REVENDICATIONS POUR L'ETAT CONTRACTANT SUIVANT: AT

**1.** Procédé de préparation des dérivés du tétrahydronaphtalène de formule générale

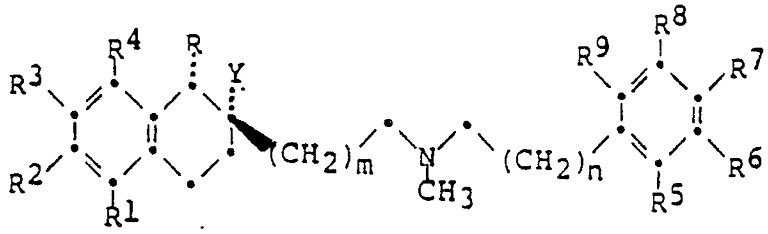

I

dans laquelle R représente l'hydrogène ou un groupe alkyle inférieur, $R^1$, $R^2$, $R^3$ et $R^4$ représentent chacun, indépendamment les uns des autres, l'hydrogène, un halogène ou un groupe alcoxy inférieur ou bien deux groupes voisins forment ensemble un groupe méthylène-dioxy ou éthylèn-dioxy, $R^5$, $R^6$, $R^7$, $R^8$ et $R^9$ représentent chacun, indépendamment les uns des autres, l'hydrogène, un halogène, un groupe alcoxy en C 1-C 10, alkylthio inférieur, (alcoxy inférieur)-alcoxy inférieur ou oméga,oméga,oméga-trifluoro-alcoxy inférieur ou bien deux groupes voisins forment ensemble un groupe méthylène-dioxy ou éthylène-dioxy, Y représente un groupe hydroxy, (alkyle inférieur)-carbonyloxy, (alcoxy inférieur)-(alkyle inférieur)-carbonyloxy, (alcoxy inférieur)-carbonyloxy, (alcoxy inférieur)-(alcoxy inférieur)-carbonyloxy, (alkylthio inférieur)-(alkyle inférieur)-carbonyloxy ou un groupe benzylcarbonyloxy éventuellement substitué, m est égal à 1 ou 2 et n est égal à 1, 2 ou 3,
à l'état de racémates et d'antipodes optiques, et de leurs sels formés par addition avec des acides acceptables pour l'usage pharmaceutique, caractérisé en ce que :

a) pour la préparation des composés de formule I dans laquelle Y représente un groupe hydroxy et les autres symboles ont les significations indiquées ci-dessus, on fait réagir un composé de formule générale

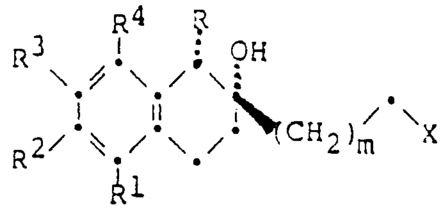

II

dans laquelle X représente un groupe éliminable et R, $R^1$, $R^2$, $R^3$, $R^4$ et m ont les significations indiquées ci-dessus, avec une amine de formule générale

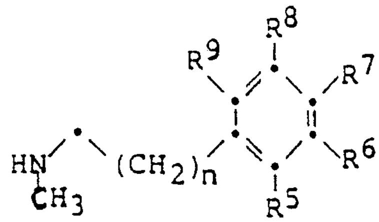

III

dans laquelle $R^5$, $R^6$, $R^7$, $R^8$, $R^9$ et n ont les significations indiquées ci-dessus, ou bien

b) pour la préparation des composés de formule I dans laquelle Y représente un groupe hydroxy et m est égal à 2, les autres symboles ayant les significations indiquées ci-dessus, on soumet un aldéhyde de formule générale

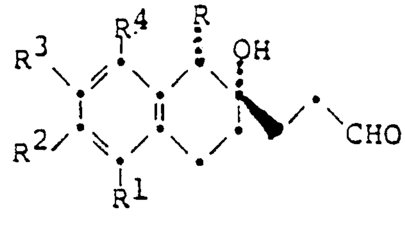

IV

dans laquelle R, $R^1$, $R^2$, $R^3$ et $R^4$ ont les significations indiquées ci-dessus,
à amination réductive par une amine de formule III ci-dessus, ou bien

c) pour la préparation des composés de formule I dans laquelle Y représente un groupe (alkyle inférieur)-carbonyloxy, (alcoxy inférieur)-(alkyle inférieur)-carbonyloxy, (alcoxy inférieur)-carbonyloxy, (alcoxy inférieur)-(alcoxy inférieur)-carbonyloxy, (alkylthio inférieur)-(alkyle inférieur)-carbonyloxy ou un groupe benzylcarbonyloxy éventuellement substitué et les autres symboles ont les significations indiquées ci-dessus, on fait réagir un composé de formule générale

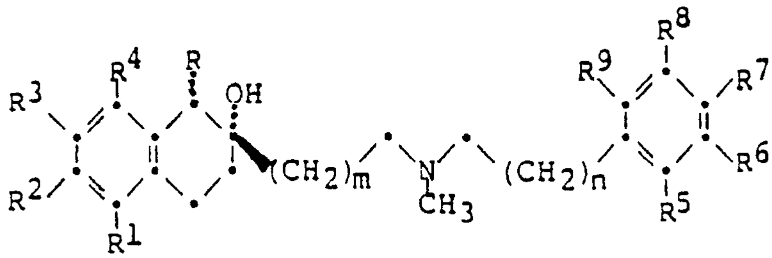

Ia

dans laquelle R, $R^1$, $R^2$, $R^3$, $R^4$, $R^5$, $R^6$, $R^7$, $R^8$, $R^9$ et m et n ont les significations indiquées ci-dessus,

avec un agent acylant apportant un groupe (alkyle inférieur)-carbonyle, (alcoxy inférieur)-(alkyle inférieur)-carbonyle, (alcoxy inférieur)-carbonyle, (alcoxy inférieur)-(alcoxy inférieur)-carbonyle, (alkylthio inférieur)-(alkyle inférieur)-carbonyle ou un groupe benzylcarbonyle éventuellement substitué, et

d) si on le désire, on résout un racémate ainsi obtenu en les antipodes optiques, et/ou

e) on convertit un composé ainsi obtenu en un sel par addition avec un acide acceptable pour l'usage pharmaceutique.

2. Procédé selon la revendication 1, dans lequel R représente un groupe alkyle inférieur.

3. Procédé selon la revendication 2, dans lequel R représente un groupe isopropyle.

4. Procédé selon l'une des revendications 1 à 3, dans lequel Y représente un groupe hydroxy, (alkyle inférieur)-carbonyloxy ou (alcoxy inférieur)-(alkyle inférieur)-carbonyloxy

5. Procédé selon la revendication 4, dans lequel Y représente un groupe hydroxy, acétyloxy ou méthoxyacétyloxy.

6. Procédé selon l'une des revendications 1 à 5, dans lequel m et n sont tous deux égaux à 1.

7. Procédé selon l'une des revendications 1 à 6, dans lequel deux des symboles $R^1$, $R^2$, $R^3$ et $R^4$ représentent l'hydrogène et les deux autres, indépendamment l'un de l'autre, l'hydrogène, des halogènes ou des groupes alcoxy inférieurs.

8. Procédé selon la revendication 7, dans lequel $R^1$ et 4 représentent tous deux l'hydrogène, $R^2$ et $R^3$ représentent chacun un groupe alcoxy inférieur ou bien $R^2$ représente un halogène et $R^3$ l'hydrogène.

9. Procédé selon l'une des revendications 1 à 8, dans lequel deux des symboles $R^5$, $R^6$, $R^7$, $R^8$ et $R^9$ représentent l'hydrogène et les trois autres représentent chacun, indépendamment les uns des autres, l'hydrogène, un halogène, un groupe alcoxy en C 1-C 10, alkylthio inférieur ou (alcoxy inférieur)-alcoxy inférieur.

10. Procédé selon la revendication 9, dans lequel $R^5$ représente l'hydrogène ou un groupe alcoxy en C 1-C 10, $R^6$ représente l'hydrogène, un groupe alcoxy en C 1-C 10 ou (alcoxy inférieur)-alcoxy inférieur, $R^7$ représente l'hydrogène, un halogène, un groupe alcoxy en C 1-C 10, alkylthio inférieur ou (alcoxy inférieur)-alcoxy inférieur et $R^8$ et $R^9$ représentent chacun l'hydrogène.

11. Procédé selon l'une des revendications 1 à 10, dans lequel R représente un groupe isopropyle, Y représente un groupe hydroxy, acétyloxy ou méthoxyacétyloxy, $R^1$,$R^4$,$R^8$ et $R^9$ représentent chacun l'hydrogène, $R^2$ et $R^3$ représentent chacun un groupe alcoxy inférieur ou bien $R^2$ représente un halogène et $R^3$ l'hydrogène, $R^5$ représente l'hydrogène ou un groupe alcoxy en C 1-C 10, $R^6$

représente l'hydrogène, un groupe alcoxy en C 1-C 10 ou (alcoxy inférieur)-alcoxy inférieur, $R^7$ représente l'hydrogène, un halogène, un groupe alcoxy en C 1-C 10, alkylthio inférieur ou (alcoxy inférieur)-alcoxy inférieur et m et n sont chacun égaux à 1.

**12.** Procédé selon la revendication 1, caractérisé en ce que l'on prépare le méthoxyacétate de 2-[2-[(3,4-diméthoxyphénéthyl)-méthylamino]-éthyl]-6-fluoro-1,2,3,4-tétrahydro-1 alpha-isopropyl-2 alpha-naphtyle.

**13.** Procédé selon la revendication 1, caractérisé en ce que l'on prépare le méthoxyacétate de 6,7-diméthoxy-2-[2-[(3,4-diméthoxyphénéthyl)-méthylamino]-éthyl]-1,2,3, 4-tétrahydro-1 alpha-isopropyl-2 alpha-naphtyle.

**14.** Procédé selon la revendication 1, caractérisé en ce que l'on prépare l'acétate de 6,7-diméthoxy-2-[2-[-(3,4-diméthoxyphénéthyl)-méthylamino]-éthyl]-1,2,3,4-tétrahydro-1 alpha-isopropyl-2 alpha-naphtyle.

**15.** Procédé selon la revendication 1, caractérisé en ce que l'on prépare le méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle.

**16.** Utilisation d'un dérivé du tétrahydronaphtalène selon l'une des revendications 1 à 15 pour la préparation de médicaments contre l'angine de poitrine, l'ischémie, les arythmies et/ou l'hypertension sanguine.

**17.** Utilisation du méthoxyacétate de (1S,2S)-2-[2-[(3,4-diméthoxyphénéthyl)-méthylamino]-éthyl]-6-fluoro-1,2,3,4-tétrahydro-1-isopropyl-2-naphtyle ou de son racémate selon la revendication 16.